# EUROPEAN PATENT APPLICATION

(11) **EP 3 091 018 A1**
(43) Date of publication of application: **09.11.2016**
(21) Application number: 15305701.3
(22) Date of filing: 07.05.2015
(51) Int. Cl.: C07D 471/08, A61K 31/439, A61K 31/04

(54) **HETEROCYCLIC COMPOUNDS AND THEIR USE IN PREVENTING OR TREATING BACTERIAL INFECTIONS**

(71) Applicant: Mutabilis, 75007 Paris (FR)
(72) Inventor: Barbion, Julien, 95110 SANNOIS (FR); Caravano, Audrey, 95880 ENGHIEN LES BAINS (FR); Chasset, Sophie, 77176 NANDY (FR); Chevreuil, Francis, 60500 CHANTILLY (FR); Lecointe, Nicolas, 75018 PARIS (FR); Ledoussal, Benoit, 22450 POMMERIT JAUDY (FR); Le Strat, Frédéric, 77380 COMBS LA VILLE (FR); Richard, Sébastien, 75020 PARIS (FR); Simon, Christophe, 94550 CHEVILLY LARUE (FR); Vomscheid, Sophie, 75014 PARIS (FR)
(74) Representative: Lavoix

(57) **Abstract**

The present invention relates to heterocyclic compounds, their process of preparation, pharmaceutical compositions comprising these compounds and use thereof, optionally in combination with other antibacterial agents and/or beta-lactam compounds, for the prevention or treatment of bacterial infections. The present invention also relates to the use of these compounds as β-lactamase inhibitors and/or as antibacterial agents.

## Description

The present invention relates to heterocyclic compounds, their process of preparation, pharmaceutical compositions comprising these compounds and use thereof, optionally in combination with other antibacterial agents and/or beta-lactam compounds, for the prevention or treatment of bacterial infections. The present invention also relates to the use of these compounds as β-lactamase inhibitors and/or as antibacterial agents.

It has been described that there is a continuous evolution of antibacterial resistance which could lead to bacterial strains against which known antibacterial compounds are inefficient.

There is thus a need to provide effective compounds and composition that can overcome bacterial antibiotic resistance.

The objective of the present invention is to provide heterocyclic compounds that can be used as antibacterial agents and/or beta-lactamase inhibitors.

An objective of the present invention is also to provide heterocyclic compounds that can be used for the prevention or for the treatment of bacterial infections.

Another objective of the present invention is to provide heterocyclic compounds that can overcome bacterial antibiotic resistance.

An objective of the invention is also to provide pharmaceutical compositions comprising such heterocyclic compounds, optionally in combination with one or more other antibacterial agent, for the prevention or for the treatment of bacterial infections and which can overcome bacterial antibiotic resistance.

Other objectives will appear throughout the description of the invention.

The present invention thus provides a compound selected from the group consisting of a compound of formula (I) wherein R¹ represents A and R² represents B and a compound of formula (I) wherein R¹ represents B and R² represents A 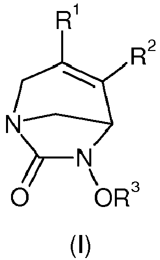 wherein
- A, unsubstituted or substituted by one or more T¹, represents a saturated, partially or totally unsaturated or aromatic 4- to 10-membered heterocycle ;
- B, represents a hydrogen atom ; a fluorine atom ; -(CH₂)ₘOQ¹ ; -(CH₂)ₘ-CN ; - (CH₂)ₘ-OC(O)Q¹ ; -(CH₂)ₘ-C(O)OQ¹ ; -(CH₂)ₘ-OC(O)OQ¹ ; -(CH₂)ₘ-OC(O)NQ¹Q²; - (CH₂)ₘ-C(O)NQ¹Q² ; -(CH₂)ₘ-C(O)ONQ¹Q² ; -(CH₂)ₘ-C(O)NQ¹OQ² ; -(CH₂)ₘ-C(O)NQ¹-NQ¹Q² ; -(CH₂)ₙ-NQ¹C(O)Q² ; -(CH₂)ₙ-NQ¹S(O)₂NQ¹Q² ; -(CH₂)ₙ-NQ¹S(O)₂Q² ; -(CH₂)ₙ-NQ¹C(O)OQ² ; -(CH₂)ₙ-NQ¹C(O)NQ¹Q² ; -(CH₂)ₙ-NQ¹Q¹ ; - (CH₂)ₙ-NH-C(NHQ³)=NQ⁴ ; -(CH₂)ₙ-NH-CH=NQ³ ; -(CH₂)ₘ-C(NHQ³)=NQ⁴ ; or an unsubstituted or substituted by one or more T², (C₁-C₃)-alkyl ; (C₁-C₃)-fluoroalkyl ; O-(C₁-C₃)-fluoroalkyl ; -(CH₂)ₘ-(C₃-C₆)-cycloalkyl ; -(CH₂)ₘ-(C₃-C₆)-cyclofluoroalkyl ;
- R³ represents -SO₃H, -CFHCOOH or -CF₂COOH;
- Q¹ and Q², identical or different, independently represent a hydrogen atom ; - (CH₂)ₚ-NHQ³ ; -(CH₂)ₚ-NH-C(NHQ³)=NQ⁴ ; -(CH₂)ₚ-NH-CH=NQ³ ; (CH₂)ₙ-C(NHQ³)=NQ⁴ ; -(CH₂)ₚ-OQ³ ; -(CH₂)ₙ-CONHQ³ ; or
   an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or
   Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ;
- Q³ and Q⁴, identical or different, independently represent a hydrogen atom or (C₁-C₃)-alkyl;
- T¹, identical or different, independently represents a fluorine atom ; -(CH₂)ₘOQ¹ ; -(CH₂)ₘ-CN ; -(CH₂)ₘ-OC(O)Q¹ ; -(CH₂)ₘ-C(O)OQ¹ ; -(CH₂)ₘ-OC(O)OQ¹ ; -(CH₂)ₘ-OC(O)NQ¹Q¹; -(CH₂)ₘ-C(O)NQ¹Q¹; -(CH₂)ₘ-C(O)ONQ¹Q²; - (CH₂)ₘ-C(O)NQ¹OQ² ; -(CH₂)ₘ-C(O)NQ¹-NQ¹Q² ; -(CH₂)ₘ-NQ¹C(O)Q² ;
   NQ¹S(O)²NQ¹Q² ; -(CH₂)ₘ-NQ¹S(O)₂Q² ; -(CH₂)ₘ-NQ¹C(O)OQ² ; -(CH₂)ₘ-NQ¹C(O)NQ¹Q² ; -(CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(CH₂)ₙ-CN ; -(X)-(CH₂)ₚ-OC(O)Q¹ ; -(X)-(CH₂)ₙ-C(O)OQ¹ ; -(X)-(CH₂)ₚ-OC(O)OQ¹ ; -(X)-(CH₂)ₚ-OC(O)NQ¹Q² ; -(X)-(CH₂)ₙ-C(O)NQ¹Q² ; -(X)-(CH₂)ₙ-C(O)ONQ¹Q² ; -(X)-(CH₂)ₙ-C(O)NQ¹OQ² ; -(X)-(CH₂)ₙ-C(O)NQ¹-NQ¹Q² ; -(X)-(CH₂)ₚ-NQ¹C(O)Q² ; -(X)-(CH₂)ₚ-NQ¹S(O)₂NQ¹Q² ; -(X)-(CH₂)ₚ-NQ¹S(O)₂Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)ₚ-NH-CH=NQ³ ; -(X)-(CH₂)ₙ-C(NHQ³)=NQ⁴ ; or
   T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; O-(C₁-C₃)-fluoroalkyl ; -(CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ;
- T², identical or different, independently represents -OH ; -NH₂ ; -CONH₂ ;
- m, identical or different, independently represents 0, 1, 2 or 3 ;
- n, identical or different, independently represents 1, 2 or 3 ;
- p, identical or different, independently represents 2 or 3 ;
- X, identical or different, independently represents O ; S ; S(O) ; S(O)₂ or N(Q³);
   wherein
- any carbon atom present within a group selected from alkyl, cycloalkyl, fluoroalkyl, cyclofluoroalkyl and heterocycle can be oxidized to form a C=O group ;
- any sulphur atom present within a heterocycle can be oxidized to form a S=O group or a S(O)₂ group ;
- any nitrogen atom present within a heterocycle or present within group wherein it is trisubstituted thus forming a tertiary amino group, can be further quaternized by a methyl group ;
and a racemate, an enantiomer, a diastereoisomer, a geometric isomer or a pharmaceutically acceptable salt thereof.

Preferably, the compound according to the invention is selected from the compounds of formulae (A) and (B) 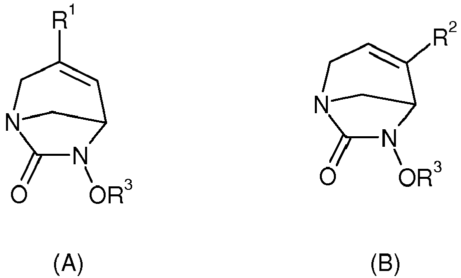 wherein R¹, R² and R³ are defined according to formula (I).

Also preferably, the compound according to the invention is selected from a compound of formula (C) 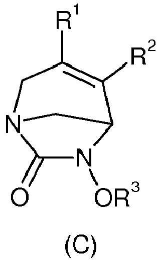 wherein R¹, R² and R³ are defined according to formula (I) provided that B does not represent a hydrogen atom.

More preferably, the compound according to the invention is selected from compounds of formulae (I*), (A*), (B*), (C*) 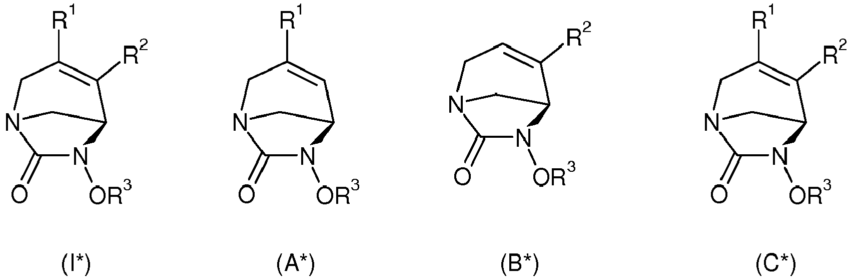 wherein R¹, R² and R³ are respectively defined according to formulae (I), (A), (B) and (C).

For the compound according to the invention, A, unsubstituted or substituted by one or more T¹, represents a saturated, partially or totally unsaturated or aromatic 4- to 10-membered heterocycle. In a preferred manner, A, unsubstituted or substituted by one or more T¹, represents a carbon-linked saturated, partially or totally unsaturated or aromatic 4- to 10-membered heterocycle.

Preferably, A, unsubstituted or substituted by one or more T¹, represents a 4-, 5- or 6-membered monocyclic heterocycle or an 8- to 10-membered bicyclic heterocycle. More preferably, A, unsubstituted or substituted by one or more T¹, represents a 4-, 5- or 6-membered monocyclic heterocycle comprising at least one nitrogen atom or an 8- to 10-membered bicyclic heterocycle comprising at least one nitrogen atom.

Equally preferably, A, unsubstituted or substituted by one or more T¹, represents a 5- or 6-membered monocyclic heterocycle comprising at least one nitrogen atom and further comprising at least one further heteroatom or heteroatomic group selected from O, S, S(O), S(O)₂ and N or an 8- to 10-membered bicyclic heterocycle comprising at least one nitrogen atom and at least one further heteroatom or heteroatomic group selected from O, S, S(O), S(O)₂ and N. Such heterocycles can advantageously comprise 1, 2 or 3 further heteroatom or heteroatomic group selected from O, S, S(O), S(O)₂ and N.

More preferably, A, unsubstituted or substituted by one or more T¹, represents a 4-, 5- or 6-membered monocyclic heterocycle and even more preferably a 4-, 5- or 6-membered monocyclic heterocycle comprising at least one nitrogen atom and possibly comprising at least one further heteroatom or heteroatomic group, for example 1,2 or 3 further heteroatom or heteroatomic group selected from O, S, S(O), S(O)₂ and N.

The invention notably provides a compound wherein A represents
- an unsubstituted or substituted by one or more T¹, saturated, partially or totally unsaturated or aromatic 4-, 5- or 6-membered heterocycle comprising at least one nitrogen atom ; or
- an unsubstituted or substituted by one or more T¹, saturated, partially or totally unsaturated or aromatic 4-, 5- or 6-membered heterocycle comprising at least one nitrogen atom and at least one further heteroatom or heteroatomic group selected from O, S, S(O), S(O)₂ and N.

Preferred compounds according to the invention are compounds of formulae (A) and (B) wherein A, unsubstituted or substituted by one or more T¹, represents a group selected from azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazol-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl. Corresponding compounds of formulae (A*) and (B*) according to the invention are equally preferred. Equally preferred compounds according to the invention are compounds of formula (C) wherein A, unsubstituted or substituted by one or more T¹, represents a group selected from azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, thiazolyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazol-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl. Corresponding compounds of formula (C*) according to the invention are equally preferred.

The term "alkyl", as used herein, refers to an aliphatic-hydrocarbon group which may be straight or branched, having 1 to 3 carbon atoms in the chain unless specified otherwise. Preferred alkyl groups have 1 or 2 carbon atoms in the chain. Specific examples of alkyl groups include, but are not limited to, methyl, ethyl, n-propyl, isopropyl. Preferably, the alkyl group is methyl or ethyl.

The term "fluoroalkyl", as used herein, refers to an alkyl group substituted with at least one fluorine atom. The term "alkyl" is as defined above. Specific examples of fluoroalkyl groups include but are not limited to trifluoromethyl, difluoromethyl, fluoromethyl.

The term "cycloalkyl" refers to a saturated monocyclic or bicyclic non-aromatic hydrocarbon ring of 3 to 6 carbon atoms, preferably 3 to 4 carbon atoms, which can comprise one or more unsaturation. Specific examples of monocyclic cycloalkyl groups include cyclopropyl, cyclobutyl, cyclopentyl, cyclohexyl. Preferably, the cycloalkyl group is cyclopropyl or cyclobutyl.

The term "fluorocycloalkyl" refers to a cycloalkyl group substituted with at least one fluorine atom. The term "cycloalkyl" is as defined above. Specific examples of fluorocycloalkyl groups include fluorocyclopropyl, difluorocyclopropyl, fluorocyclobutyl, difluorocyclobutyl.

The term "heterocycle", as used herein and without contrary definition specifically mentioned, either alone or in combination with another radical, refers to a monocyclic saturated, partially or totally unsaturated or aromatic hydrocarbon radical, preferably to a 4- to 10-membered hydrocarbon radical, comprising at least one heteroatom, such as N, O, S, S(O) or S(O)₂. Preferably, the heterocycle is a monocyclic saturated, partially or totally unsaturated or aromatic hydrocarbon radical, preferably a 4- to 6-membered hydrocarbon radical, comprising at least one nitrogen atom and at least one further heteroatom, such as N, O, S, S(O) or S(O)₂. The carbon atoms of the heterocycle can also be oxidized to form a C(O) group. Suitable heterocycles are also disclosed in the Handbook of Chemistry and Physics, 76th Edition, CRC Press, Inc., 1995-1996, pages 2-25 to 2-26. Exemplary heterocycle groups include but are not limited to azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, thiazolyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazol-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl. Preferably, in the compounds according to the invention, the heterocycle is linked to the structure of the compounds by a carbon atom of the heterocycle (also said carbon-linked heterocycle).

Moreover some compounds according to this invention may contain a basic amino group and thus may form an inner zwitterionic salt (or zwitterion) with the acidic group (R³)-OSO₃H, -OCFHCO₂H or-OCF₂CO₂H and such inner zwitterionic salts are also included in this invention.

The expression "optionally substituted" means "non-substituted or substituted by chemical groups that are further defined" or "unsubstituted or substituted chemical groups that are further defined".

The term "racemate" is employed herein to refer to an equal amount of two specific enantiomers.

The term "enantiomer" is employed herein to refer to one of the two specific stereoisomers which is a non-superimposable mirror image with one other but is related to one other by reflection.

The compounds according to the invention may include one or more asymmetric carbon atoms and may thus exist in the form of optical isomers as well as in the form of racemic or non-racemic mixtures thereof. The compounds according to the invention can be utilized as a single isomer or as a mixture of stereochemical isomeric forms. Diastereoisomers, *i.e.,* non-superimposable stereochemical isomers can be separated by conventional means such as chromatography, distillation, crystallization or sublimation. The optical isomers (enantiomers) can be obtained by using optically active starting materials, by resolution of the racemic mixtures according to conventional processes, for example by formation of diastereoisomeric salts by treatment with an optically active acid or base or by using chiral chromatography column.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention can be synthesized from the parent compound which comprises a basic or an acidic moiety, by conventional chemical methods. Furthermore, the expression "pharmaceutically acceptable salt" refers to relatively nontoxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate, palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labelled compounds wherein one or more atoms is replaced by an atom having the same atomic number, but an atomic mass or mass number different from the atomic mass or mass number usually found in nature. Examples of isotopes suitable for inclusion in the compounds described above and are not limited to ²H, ³H, ¹¹C, ¹³C, ¹⁴C, ¹⁸F, ¹⁹F, ¹³N, ¹⁵N, ³³S, ³⁴S, ³⁵S, ³⁶S, ¹⁷O or ¹⁸O. Isotopically-labelled compounds are useful in drug and/or substrate tissue distribution studies. Substitution with heavier isotopes such as deuterium (²H) affords greater metabolic stability (for example increased *in vivo* half-life or reduced dosage requirements). Isotopically-labelled compounds are prepared by any suitable method or by processes using an appropriate isotopically-labelled reagent in replacement of the non-labelled reagent otherwise employed.

The invention provides compounds having antibacterial properties and/or compounds acting as β-lactamase inhibitors.

The invention also provides a process for the preparation of a compound according to the invention. In particular the invention provides a process for the preparation of compound selected within the compounds of formulae (I), (A), (B), (C), (I*), (A*), (B*), (C*) according to the invention.

A general process according to the invention is represented in scheme 1 wherein R² represents various substituents.

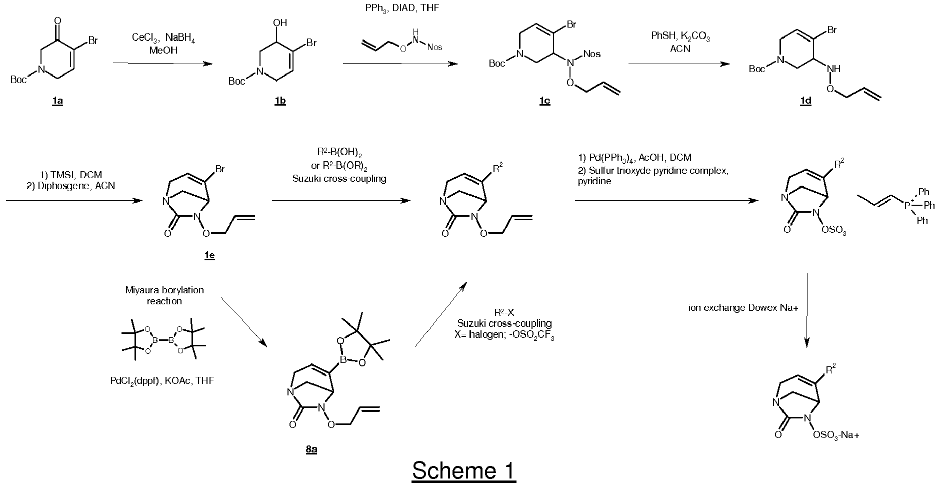

The process of scheme 1 can be adapted for preparing further compounds according to the invention. Further processes for the preparation of compounds according to the invention can be derived from the process of scheme 1.

The invention also provides particular processes represented in the schemes of the experimental part that is provided herein for the preparation of compounds according to the invention wherein R¹, R² and R³ represent various substituents. These processes can also be adapted for preparing further compounds according to the invention. Further processes for the preparation of compounds according to the invention can be derived from these processes.

The invention also provides the use of the compounds according to the invention in the control of bacteria. The compound according to the invention is then usually used in combination with at least one pharmaceutically acceptable excipient.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present invention also provides a composition, preferably a pharmaceutical composition, comprising at least one compound according to the invention in mixture with a pharmaceutically acceptable excipient. The composition according to the invention may thus comprise at least one compound selected from compounds of formulae (I), (A), (B), (C), (I*), (A*), (B*) and (C*) in mixture with a pharmaceutically acceptable excipient.

The composition according to the invention can further comprise at least one or more antibacterial agent(s), preferably at least one of these antibacterial agents is a beta-lactam.

The term "beta-lactam" or "β-lactam" refers to antibacterial compounds comprising a β-lactam unit, *i.e.* a β-lactam chemical group or moiety.

The term "pharmaceutically acceptable carrier" or "pharmaceutically acceptable excipient" is employed for any excipient, solvent, dispersion medium, absorption retardant, diluent or adjuvant etc., such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium crosscarmellose, glucose, gelatine, starch, lactose, dicalcium phosphate, sucrose, kaolin, magnesium carbonate, wetting agents, emulsifying agents, solubilizing agents, sterile water, saline, pH buffers, non-ionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed., Pergamon press., 1990). Except insofar as any conventional media or adjuvant is incompatible with the active ingredient according to the invention, its use in the therapeutic compositions is contemplated.

The expression "antibacterial agent" as used herein, refers to any substance, compound or their combination capable of inhibiting, reducing or preventing growth of bacteria, inhibiting or reducing ability of bacteria to produce infection in a subject, or inhibiting or reducing ability of bacteria to multiply or remain infective in the environment, or decreasing infectivity or virulence of bacteria.

The antibacterial agent can be selected among the following families: aminoglycosides, beta-lactams, glycylcyclines, tetracyclines, quinolones, fluoroquinolones, glycopeptides, lipopeptides, macrolides, ketolides, lincosamides, streptogramins, oxazolidinones and polymyxins alone or in mixture. Preferably, the further antibacterial agent is selected among the beta-lactam families, and more preferably among penicillin, cephalosporins, penems, carbapenems and monobactam, alone or in mixture.

Among the penicillin the antibacterial agent is preferably selected in the group consisting of amoxicillin, ampicillin, azlocillin, mezocillin, apalcillin, hetacillin, bacampicillin, carbenicillin, sulbenicillin, temocillin, ticarcillin, piperacillin, mecillinam, pivmecillinam, methicillin, ciclacillin, talampacillin, aspoxicillin, oxacillin, cloxacillin, dicloxacillin, flucloxacillin, nafcillin, and pivampicillin, alone or in mixture.

Among the cephalosporin, the antibacterial agent is preferably selected in the group consisting of cefatriazine, cefazolin, cefoxitin, cephalexin, cephradine, ceftizoxime, cephacetrile, cefbuperazone, cefprozil, ceftobiprole, ceftobiprole medocaril, ceftaroline, ceftaroline fosaminyl, cefalonium, cefminox, ceforanide, cefotetan, ceftibuten, cefcapene pivoxil, cefditoren pivoxil, cefdaloxime cefroxadine, ceftolozane and S-649266, cephalothin, cephaloridine, cefaclor, cefadroxil, cefamandole, cefazolin, cephalexin, cephradine, ceftizoxime, cephacetrile, cefotiam, cefotaxime, cefsulodin, cefoperazone, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidine, cefixime, ceftibuten, cefdinir, cefpodoxime axetil, cefpodoxime proxetil, cefteram pivoxil, cefetamet pivoxil, cefcapene pivoxil, cefditoren pivoxil, cefuroxime, cefuroxime axetil, loracarbef, and latamoxef, alone or in mixture. Among the carbapenem, the antibacterial agent is preferably selected in the group consisting of imipenem, doripenem, meropenem, biapenem, ertapenem and panipenem, alone or in mixture.

Among the monobactam, the antibacterial agent is preferably selected in the group consisting of aztreonam, tigemonam, carumonam, BAL30072 and nocardicin A, alone or in mixture.

The present invention also provides a kit comprising:
▪ a pharmaceutical composition according to the invention, and
▪ at least one other composition comprising one or more antibacterial agents, preferably at least one of these antibacterial agents is a beta-lactam.

The two compositions can each be prepared separately with one specific pharmaceutically acceptable carrier, and can then be mixed, especially extemporaneously.

The present invention also refers to a compound selected within the compounds of formulae (I), (A), (B), (C), (I*), (A*), (B*) and (C*) according to the invention for its use as a medicine.

The present invention also refers to a compound selected within the compounds of formulae (I), (A), (B), (C), (I*), (A*), (B*) and (C*) according to the invention for its use for the preparation of a medicine.

The present invention also refers to a compound selected within the compounds of formulae (I), (A), (B), (C), (I*), (A*), (B*) and (C*) according to the invention for its use as an antibacterial agent.

The present invention also refers to the use of a compound selected within the compounds of formulae (I), (A), (B), (C), (I*), (A*), (B*) and (C*) according to the invention or to the use of a pharmaceutical composition according to the invention for the preparation of an antibacterial agent comprising medicine.

The present invention also refers to the use of a compound selected within the compounds of formulae (I), (A), (B), (C), (I*), (A*), (B*) and (C*) according to the invention or to the use of a pharmaceutical composition according to the invention for the preparation of a beta-lactamase inhibitor comprising medicine.

The present invention also refers to the use of a compound selected within the compounds of formulae (I), (A), (B), (C), (I*), (A*), (B*) and (C*) according to the invention or to the use of a pharmaceutical composition according to the invention for the preparation of a medicine comprising an antibacterial agent and a beta-lactamase inhibitor.

The present invention also refers to the use of a compound selected within the compounds of formulae (I), (A), (B), (C), (I*), (A*), (B*) and (C*) according to the invention or to the use of a pharmaceutical composition according to the invention or to the use of a kit according to the invention for the treatment or for the prevention of at least one bacterial infection.

The present invention also refers to the use of a compound selected within the compounds of formulae (I), (A), (B), (C), (I*), (A*), (B*) and (C*) according to the invention or to the use of a pharmaceutical composition according to the invention or to the use of a kit according to the invention for the preparation of a medicine useful in the treatment or in the prevention of at least one bacterial infection.

The terms "prevention", "prevent" and "preventing" as used herein are intended to mean the administration of a compound or composition according to the invention in order to prevent infection by bacteria or to prevent occurrence of related infection and/or diseases. The terms "prevention", "prevent" and "preventing" also encompass the administration of a compound or composition according to the present invention in order preventing at least one bacterial infection, by administration to a patient susceptible to be infected, or otherwise at a risk of being infected by this bacteria.

The terms "treatment", "treat" and "treating" as used herein are intended to mean in particular the administration of a treatment comprising a compound or composition according to the invention to a patient suffering from an infection. The terms "treatment", "treat" and "treating" as used herein, also refer to administering a compound or composition according to the invention, optionally in combination with one or more further antibacterial agent, in order:
▪ to reduce or to eliminate either bacterial infection or one or more symptoms associated with a bacterial infection, or
▪ to retard the progression of a bacterial infection or of one or more symptoms associated with a bacterial infection, or
▪ to reduce the severity of a bacterial infection or of one or more symptoms associated with a bacterial infection, or
▪ to suppress the clinical manifestation of a bacterial infection, or
▪ to suppress the manifestation of adverse symptoms caused by a bacterial infection.

The expression "infection" or "bacterial infection" as used herein, include the presence of bacteria, in or on a subject, which, if its growth were inhibited, would result in a benefit to the subject. As such, the term "infection" or "bacterial infection" in addition to referring to the presence of bacteria also refer to normal flora, which is not desirable. The term "infection" includes infection caused by bacteria. Examples of such bacterial infections are urinary tract infection (UTI), kidney infections (pyelonephritis), gynecological and obstetrical infections, respiratory tract infection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, bacteremia.

The term "growth" as used herein, refers to the growth of one or more microorganisms and includes reproduction or population expansion of a microorganism, such as bacteria. The term also includes maintenance of on-going metabolic processes of a microorganism, including processes that keep the microorganism alive.

According to the invention, bacteria are chosen amongst gram-positive bacteria or gram-negative bacteria, preferably gram-negative bacteria. According to the invention, bacteria can be also chosen among bacteria producing "beta-lactamase" or "β**-**lactamase". These bacteria are well known by the person skilled in the art. The term "beta-lactamase" or "β-lactamase" as used herein, refers to any enzyme or protein or any other substance that is able to break down a beta-lactam ring. The term "beta-lactamase" or "β-lactamase" includes enzymes that are produced by bacteria and that have the ability to hydrolyze, either partially or completely, the beta-lactam ring present in a compound such as an antibacterial agent.

Among the gram-positive bacteria, the bacteria according to the invention is preferably chosen among *Staphylococcus, Streptococcus, Staphylococcus species* (including *Staphylococcus aureus, Staphylococcus epidermidis*)*, Streptococcus species* (including *Streptococcus pneumonia, Streptococcus agalactiae*), *Enterococcus species* (including *Enterococcus faecalis* and *Enterococcus faecium*).

Among the gram-negative bacteria, the bacteria according to the invention is preferably chosen among *Acinetobacter* species (including *Acinetobacter baumannii*), *Citrobacter* species, *Escherichia* species (including *Escherichia coli*), *Haemophilus influenza, Morganella morganii, Klebsiella* species (including *Klebsiella pneumonia*), *Enterobacter* species (including *Enterobacter cloacae*), *Neisseria gonorrhoeae, Burkholderia* species (including *Burkholderia cepacia*), (*Proteus* species (including *Proteus mirabilis*), *Serratia* species (including *Serratia marcescens*), *Pseudomonas aeruginosa.*

The invention thus preferably refers to a compound selected within the compounds of formulae (I), (A), (B), (C), (I*), (A*), (B*) and (C*) according to the invention or to a pharmaceutical composition according to the invention or to a kit according to the invention for its use for the treatment or for the prevention of a bacterial infection, preferably caused by bacteria producing one or more beta-lactamases. Preferably, the bacteria are chosen amongst gram-positive bacteria or gram-negative bacteria, more preferably gram-negative bacteria.

The present invention also refers to the use of a compound selected within the compounds of formulae (I), (A), (B), (C), (I*), (A*), (B*) and (C*) according to the invention or to a pharmaceutical composition according to the invention for the preparation of a medicine for the treatment or for the prevention of a bacterial infection, preferably caused by bacteria producing one or more beta-lactamases. Preferably, the bacteria are chosen amongst gram-positive bacteria or gram-negative bacteria, more preferably gram-negative bacteria.

The present invention also refers to a kit according to the invention, for its simultaneous, separated or sequential administration to a patient in need thereof in the treatment or in the prevention of bacterial infections, preferably caused by bacteria producing one or more beta-lactamases. Preferably, the bacteria are chosen amongst gram-positive bacteria or gram-negative bacteria, more preferably gram-negative bacteria.

The present invention also refers to a compound selected within the compounds of formulae (I), (A), (B), (C), (I*), (A*), (B*) and (C*) according to the invention for its use in combination with one or more further antibacterial agents, preferably at least one of the further antibacterial agents being a beta lactam compound, for the treatment or for the prevention of bacterial infections, preferably caused by bacteria producing one or more beta-lactamases. Preferably, the bacteria are chosen amongst gram-positive bacteria or gram-negative bacteria, more preferably gram-negative bacteria, and wherein a compound selected within the compounds of formulae (I), (A), (B), (C), (I*), (A*), (B*) and (C*) according to the invention and the further antibacterial agent are administered simultaneously, separately or sequentially.

The present invention also refers to the use of a compound selected within the compounds of formulae (I), (A), (B), (C), (I*), (A*), (B*) and (C*) according to the invention or of a pharmaceutical composition according to the invention or of a kit according to the invention for the prevention or for the treatment of bacterial infections, preferably of a bacterial infection, preferably caused by bacteria producing one or more beta-lactamases. Preferably, the bacteria are chosen amongst gram-positive bacteria or gram-negative bacteria, more preferably gram-negative bacteria.

The present invention also relates to a method for the treatment or prevention of bacterial infections, preferably caused by bacteria producing one or more beta-lactamases comprising the administration of a therapeutically effective amount of a compound selected within the compounds of formulae (I), (A), (B), (C), (I*), (A*), (B*) and (C*) according to the invention, or of a pharmaceutical composition according to the invention or of a kit according to the invention to a patient in need thereof. Preferably, the bacteria are chosen amongst gram-positive bacteria or gram-negative bacteria, more preferably gram-negative bacteria.

The term "patient" means a person or an animal at risk of being infected by bacteria or, a person or an animal being infected by bacteria, preferably by gram-positive and by gram-negative bacteria, more preferably by gram-negative bacteria. As used herein, the term "patient" refers to a warm-blooded person or animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical or family history or biological and diagnostic tests, those subjects who are in need of such a treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician. The amount of a compound according to the invention which constitutes a "therapeutically effective amount" will vary, notably depending on the compound itself and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a "therapeutically effective amount" can be determined by one of ordinary skilled in the art having regard to its own knowledge, and this disclosure. Preferably, the compound according to the invention is administered in an amount comprised between 0.1 to 30 g per day.

The compound according to the invention may be provided in an aqueous physiological buffer solution for parenteral administration. The compound of the present invention is also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described herein. The compound provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches.

The pharmaceutical composition may be conveniently administered in unit dosage form and may be prepared by any method well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.

Preferred formulations include pharmaceutical compositions wherein a compound according to the present invention is formulated for oral or parenteral administration.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings and flavorings. In addition, the active compounds may be incorporated into fast dissolved, modified-release or sustained-release preparations and formulations, and wherein such sustained-release formulations are preferably bi-modal. Preferred tablets contain lactose, corn-starch, magnesium silicate, crosscarmellose sodium, povidone, magnesium stearate or talc in any combination. Liquid preparations for parenteral administration include sterile aqueous or non-aqueous solutions, suspensions and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compound. Intravenous vehicles can include fluid and nutrient replenishers, electrolyte replenishers, such as those based on Ringer's dextrose, and the like. Other potentially useful parenteral delivery systems for the active compound include ethylene-vinyl acetate copolymer particles, osmotic pumps, implantable infusion systems and liposomes.

Alternative modes of administration include formulations for inhalation, which include such means as dry powder, aerosol, or drops. They may be aqueous solutions comprising, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations.

Formulations suitable for transdermal administration can be presented as discrete patches and can be lipophilic emulsions or buffered, aqueous solutions, dissolved and/or dispersed in a polymer or an adhesive.

### Examples

The following examples are provided for the purpose of illustrating the present invention and by no means should be interpreted to limit the scope of the present invention.

The first part represents the preparation of the compounds (intermediates and final compounds) whereas the second part describes the evaluation of antibacterial activity of compounds according to the invention.

### Preparation of the compounds and biological activity:

Abbreviations or symbols used herein include:
   - ACHN:: 1,1'-azobis(cyclohexanecarbonitrile)
   - ACN:: acetonitrile
   - AcOH:: acetic acid
   - Bn:: benzyl
   - Boc:: *tert-*butoxycarbonyl
   - Boc₂O:: *tert*-butoxycarbonyl anhydride
   - bs:: broad singlet
   - CFU:: colony-forming units
   - CLSI:: clinical laboratory standards institute
   - d:: doublet
   - DBU:: 1,8-diazabicyclo[5.4.0]undec-7-ene
   - DCM:: dichloromethane
   - dd:: doubled doublet
   - ddd :: doubled doubled doublet
   - dt :: doubled triplet
   - DTA:: di-*tert*-butylazodicarboxylate
   - DEAD:: diethyl azodicarboxylate
   - DIAD:: diisopropyl azodicarboxylate
   - DIPEA:: diisopropylethylamine
   - DMF:: *N,N*-dimethylformamide
   - DMAP:: 4-dimethylaminopyridine
   - DMSO:: dimethylsulfoxide
   - EtOAc:: ethyl acetate
   - Et₂O:: diethyl ether
   - h:: hours
   - IC₅₀:: concentration of inhibitor responsible for 50% of inhibition
   - KOAc:: potassium acetate
   - m :: massif
   - min:: minutes
   - MeOH:: methanol
   - MIC:: minimum inhibitory concentration
   - MS:: mass spectrometry
   - MTBE:: methyl *tert*-butyl ether
   - NBS:: *N*-bromosuccinimide
   - Ni(COD)₂:: Bis(1,5-cyclooctadiene)nickel(0)
   - NMR:: nuclear magnetic resonance spectroscopy
   - Nos:: nosyl, nitrobenzenesulfonyl
   - Pd(dppf)Cl₂:: [1,1'-bis(diphenylphosphino)ferrocene]dichloropalladium
   - Pd(PPh₃)₄:: tetrakis(triphenylphosphine)palladium(0)
   - PEPPSI:: [1,3-bis(2,6-diisopropylphenyl)imidazol-2-ylidene](3-chloropyridyl)palladium(ll) dichloride
   - PPh₃:: triphenylphosphine
   - Ppm:: parts per million
   - q:: quadruplet
   - qd:: doubled quadruplet
   - rt:: room temperature
   - s:: singlet
   - t:: triplet
   - TEA:: trimethylamine
   - TFA:: trifluoroacetic acid
   - THF:: tetrahydrofuran
   - TLC:: thin layer chromatography
   - TMSI:: iodotrimethylsilane

### Example 1: synthesis of (7-oxo-4-pyrazol-4-yl-1,6-diaza-bicyclo[3.2.1]oct-3-en-6-yl) sulfate sodium salt

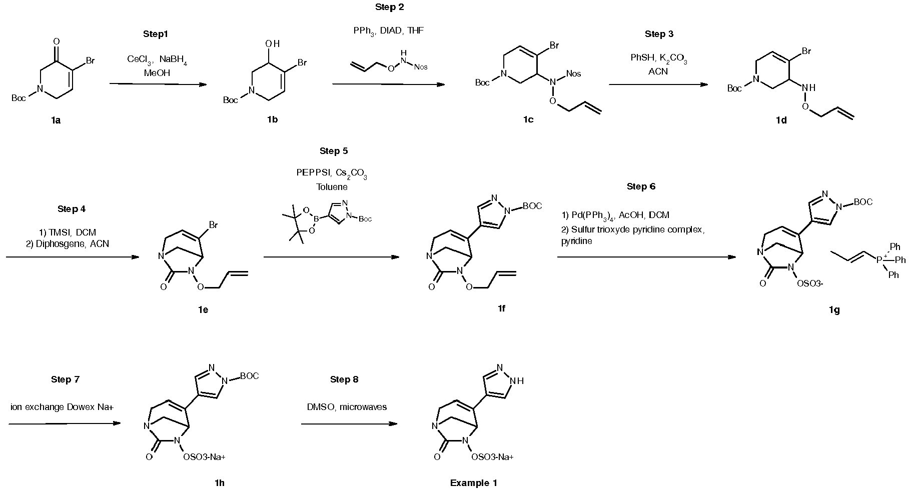

### Step 1: preparation of intermediate tert-butyl 4-bromo-3-hydroxy-3,6-dihydro-2H-pyridine-1-carboxylate (1b)

In a 250 mL round bottom flask under inert atmosphere, *tert*-butyl 4-bromo-3-oxo-3,6-dihydro-2*H*-pyridine-1-carboxylate (1 a, prepared according to Tetrahedron Lett., 1994, 35, 3589-3592) (2.875 g, 10.41 mmol) was diluted with anhydrous MeOH (50 mL). The clear solution was cooled down to 0 °C with an ice bath and heptahydrate cerium chloride (III) was then added (4.46 g, 11.97 mmol). NaBH₄ (0.492 g, 13.01 mmol) was added by portion over 20 min. The resulting suspension was stirred until complete conversion of the starting material. Reaction mixture was filtered on celite, washed with MeOH (50 mL). The filtrate was diluted with EtOAc (250 mL) and cooled down to 0 °C. 0.1 M aqueous hydrochloric acid was added to reach pH 5-6. Aqueous layer was extracted with EtOAc (3x75 mL). Combined organic layers were dried over sodium sulfate, filtered and concentrated under reduced pressure. Crude residue was purified by flash chromatography on silica gel (heptane/EtOAc 60/40) to give desired *tert*-butyl 4-bromo-3-hydroxy-3,6-dihydro-2*H*-pyridine-1-carboxylate (1 b) (2.85 g, 10.24 mmol, 98%).
MS *m*/*z* ([M-(*tert*-butyl)+H]⁺) 222-224.
¹H NMR (400 MHz, CDCl₃): *δ* (ppm) 1.49 (s, 9H), 2.50 (bs, 1H), 3.66 (dd, J= 13.7/4.0 Hz, 1 H), 3.73-3.90 (m, 2H), 4.08 (d, *J=* 18.3 Hz, 1 H), 4.24 (bs, 1 H), 6.20 (bs, 1H).

### Step 2: preparation of intermediate tert-butyl 3-[allyloxy-(4-nitrophenyl)sulfonyl-amino]-4-bromo-3,6-dihydro-2H-pyridine-1-carboxylate (1c)

Under inert atmosphere, to a solution of *tert*-butyl 4-bromo-3-hydroxy-3,6-dihydro-2*H* pyridine-1-carboxylate (1 b) (2.85 g, 10.25 mmol) in THF (100 mL) was added *N-*(allyloxy)-2-nitrobenzenesulfonamide (3.97 g, 15.37 mmol), PPh₃ (8.06 g, 30.74 mmol) and DIAD (6.05 mL, 30.74 mmol). The pale yellow solution turned to an orange suspension. The reaction was stirred for 12 h at rt and was then concentrated under reduced pressure. The pale orange residue was taken up in DCM (50 mL) and dried under reduced pressure to give an orange oil. Purification by flash chromatography on silica gel (toluene/Et₂O 85/15) gave pure *tert*-butyl 3-[allyloxy-(4-nitrophenyl)sulfonyl-amino]-4-bromo-3,6-dihydro-2*H-*pyridine-1-carboxylate (1c) (3.73 g, 7.20 mmol, 71%) as a pale yellow oil.
MS *m*/*z* ([M-(*tert*-Butyl)+H]⁺) 462-464.
¹H NMR (300 MHz, CDCl₃): *δ* (ppm) 1.39 (bs, 9H), 3.11-3.42 (m, 1H), 3.64 (d, *J=* 18.8 Hz, 1 H), 3.93-4.56 (m, 4H), 4.64 (bs, 1 H), 5.17-5.33 (m, 2H), 5.75-5.92 (m, 1 H), 6.43 (bs, 1 H), 7.64 (d, *J* = 7.1 Hz, 1 H), 7.72-7.87 (m, 2H), 8.18 (d, *J* = 7.1 Hz, 1H).

### Step 3: preparation of intermediate tert-butyl 3-allyloxyamino-4-bromo-5,6-dihydropyridine-1 (2H)-carboxylate (1d)

In a 100 mL round bottom flask under inert atmosphere, *tert*-butyl 3-[allyloxy-(4-nitrophenyl)sulfonyl-amino]-4-bromo-3,6-dihydro-2*H-*pyridine-1-carboxylate (1c) (3.72 g, 7.18 mmol) was diluted at rt with ACN (70 mL). To the clear yellow solution were added thiophenol (3.68 mL, 35.88 mmol) and K₂CO₃ (7.44 g, 53.82 mmol). The resulting yellow suspension turned to orange and was stirred for 12 h. The mixture was filtered on 0.45 µm PTFE membrane and the filtrate was concentrated under reduced pressure. The crude compound was purified by flash chromatography on silica gel (toluene/acetone 98/2) to give desired *tert*-butyl 3-allyloxyamino-4-bromo-5,6-dihydropyridine-1(2*H*)-carboxylate (1d) (1.84 g, 5.51 mmol, 77%).
MS *m*/*z* ([M+H]⁺) 331-333.
¹H NMR (300 MHz, CDCl₃): δ (ppm) 1.52 (bs, 9H), 3.32 (dd, *J =* 13.6/ 3.7 Hz, 1 H), 3.57-3.83 (m, 2H), 4.05-4.40 (m, 4H), 5.17-5.39 (m, 2H), 5.82 (bs, 1 H), 5.90-6.08 (m, 1 H), 6.26 (bs, 1H).

### Step 4: preparation of intermediate 6-allyloxy-4-bromo-1,6-diaza-bicyclo[3.2.1]oct-3-en-7-one (1e)

In a 250 mL round bottom flask under inert atmosphere, *tert*-butyl 3-allyloxyamino-4-bromo-5,6-dihydropyridine-1(2*H*)-carboxylate (1d) (1.84 g, 5.51 mmol) was diluted in anhydrous DCM (150 mL). TMSI (1.23 mL, 8.26 mmol) was then slowly added over 10 min. The resulting yellow suspension was stirred at rt for 10 min until complete conversion of the starting material. The reaction mixture was cooled down to 0 °C with an ice bath and quenched with MeOH (10 mL). The resulting pale yellow solution was concentrated to dryness under reduced pressure to give a brown gum (2.09 g) containing crude 3-allyloxyamino-4-bromo-5,6-dihydropyridine which was engaged without further purification in the next step.

In a 500 mL round bottom flask under inert atmosphere, crude 3-allyloxyamino-4-bromo-5,6-dihydropyridine was diluted with anhydrous ACN (150 mL). The yellow solution was cooled down to 0 °C and TEA (3.07 mL, 22.03 mmol) was added. Diphosgene (366 µL, 3.03 mmol) diluted in ACN (60 mL) was slowly added over 1 h. The reaction mixture was stirred at 0 °C for 1 hour until complete conversion of the starting material. The pale brown solution was concentrated under reduced pressure and the brown residue was taken up in EtOAc (200 mL), then washed with saturated aqueous sodium hydrogenocarbonate solution (50 mL) and brine (50 mL). The organic layer was dried over sodium sulfate, filtered and dried under reduced pressure. The crude material was purified by flash chromatography on silica gel (toluene/EtOAc 85/15) and gave pure 6-allyloxy-4-bromo-1,6-diaza-bicyclo[3.2.1]oct-3-en-7-one (1 e) as a pale yellow solid (638 mg, 2.46 mmol, 45% over 2 steps).
MS *m*/*z* ([M+H]⁺) 259-261.
¹H NMR (400 MHz, CDCl₃): *δ* (ppm) 3.20 (d, *J =* 10.9 Hz, 1 H), 3.53 (dd, *J =* 10.9/3.1 Hz, 1 H), 3.72 (dd, *J =* 18.0/ 2.1 Hz, 1 H), 3.85 (dd, *J =* 18.0/ 3.4 Hz, 1 H), 4.05-4.09 (m, 1 H), 4.39-4.56 (m, 2H), 5.31-5.46 (m, 2H), 6.00-6.13 (m, 2H).

### Step 5: preparation of intermediate tert-butyl 4-(6-allyloxy-7-oxo-1,6-diazabicyclo [3.2.1]oct-3-en-4-yl)pyrazole-1-carboxylate (1f)

In a 5 mL sealed tube under inert atmosphere, 6-allyloxy-4-bromo-1,6-diaza-bicyclo[3.2.1]oct-3-en-7-one (1e) (79.3 mg, 0.306 mmol) was diluted at rt with anhydrous toluene (3.1 mL). Anhydrous CsCO₃ (133 mg, 0.408 mmol) and *tert*-butyl 4-(4,4,5,5-tetramethyl-1,3,2-dioxaborolan-2-yl)pyrazole-1-carboxylate (60 mg, 0.204 mmol) were added. Argon was bubbled through the resulting white suspension for 10 min and PEPPSI catalyst was then added (10.4 mg, 0.015 mmol). The mixture was heated under microwaves at 100 °C for 50 min to reach maximal conversion of the starting material. The medium was filtered through 0.45 µm membrane, the brown filtrate was diluted with EtOAc (10 mL) and washed with brine (2x2 mL). Aqueous layers were extracted with EtOAc (2x2 mL). Organic layers were dried over sodium sulfate, filtered and concentrated under reduced pressure to give crude material (148 mg). Further purification by flash chromatography on silica gel (DCM/EtOAc 100/0 to 70/30) gave pure *tert*-butyl 4-(6-allyloxy-7-oxo-1,6-diazabicyclo [3.2.1]oct-3-en-4-yl)pyrazole-1-carboxylate (1f) as a yellowish oil (34 mg, 0.098 mmol, 32%).
MS *m*/*z* ([M+H]⁺) 347.
¹H NMR (300 MHz, CDCl₃): *δ* (ppm) 1.51 (s, 9H), 3.04 (d, *J=* 10.8 Hz, 1H), 3.45 (dd, *J* = 10.9/ 2.8 Hz, 1 H), 3.68 (dd, *J =* 18.8/ 2.0 Hz, 1 H), 3.80 (dd, *J =* 18.8/ 2.8 Hz, 1 H), 3.93 (bd, *J =* 2.7 Hz, 1 H), 4.21-4.38 (m, 2H), 5.13-5.28 (m, 2H), 5.68-5.72 (m, 1 H), 5.80-5.95 (m, 1 H), 7.58 (d, *J* = 0.9 Hz, 1 H), 7.87 (bs, 1H).

### Step 6: preparation of intermediate tert-butyl 4-(7-oxo-1,6-diazabicyclo[3.2.1]oct-3-en-4-yl)pyrazole-1-carboxylate sulfate triphenyl-(propenyl)-phosphonium salt (1g)

To a solution of *tert*-butyl 4-(6-allyloxy-7-oxo-1,6-diazabicyclo [3.2.1]oct-3-en-4-yl)pyrazole-1-carboxylate (1f) (27 mg, 0.078 mmol) in anhydrous DCM (1 mL) were added glacial AcOH (9 µL, 0.156 mmol) and Pd(PPh₃)₄ (45 mg, 0.039 mmol). After 45 min of stirring at rt, pyridine (1 mL) and sulfur trioxide pyridine complex (62 mg, 0.390 mmol) were added to the reaction mixture. The resulting white suspension was protected from light and stirred overnight until the reaction was completed. The suspension was filtered, the solids were washed with DCM (3 x 5 mL), the filtrate was concentrated under *vacuum* and then purified by flash chromatography on silica gel (DCM/acetone: 100/0 to 25/75) to afford (1g) (35 mg) as a triphenyl-(propenyl)-phosphonium salt.
MS *m*/*z* ([M-H]⁻) 385.
MS *m*/*z* ([M+H]⁺) 303 (triphenyl-propenyl-phosphonium).

### Step 7: preparation of intermediate tert-butyl 4-(7-oxo-1,6-diaza-bicyclo[3.2.1]oct-3-en-4-yl)pyrazole-1-carboxylate sulfate sodium salt (1h)

*Tert*-butyl 4-(7-oxo-1,6-diaza-bicyclo[3.2.1]oct-3-en-4-yl)pyrazole-1-carboxylate ester sulfonate triphenyl-(propenyl)-phosphonium salt (1g) (35 mg) dissolved in water (200 µL) was applied on a Dowex sodium form column (Dowex® 50WX8 hydrogen form stored with an aqueous solution of 2N NaOH and washed until neutral pH with water). The fractions containing the desired compound were combined, frozen and lyophilized to afford *tert-*butyl 4-(7-oxo-1,6-diaza-bicyclo[3.2.1]oct-3-en-4-yl)pyrazole-1-carboxylate ester sulfonate sodium salt (1 h) (17.5 mg, 0.265 mmol, 55% over 2 steps) as a white amorphous solid.
MS *m*/*z* ([M-H]⁻) 385.

### Step 8: preparation of (7-oxo-4-pyrazol-4-yl-1,6-diaza-bicyclo[3.2.1]oct-3-en-6-yl) sulfate sodium salt (Example 1)

In a 2 mL sealed tube, mono *tert*-butyl 4-(7-oxo-1,6-diaza-bicyclo[3.2.1]oct-3-en-4-yl)pyrazole-1-carboxylate ester sulfonate sodium salt (1h) (14.4 mg, 0.035 mmol) was diluted with DMSO (600 µL). The resulting solution was saturated with argon and heated under microwaves at 140 °C for 5 min. The solution turned yellow and was directly frozen and lyophilized. The residue was taken up with water (2 mL), filtered over 0.20 µm membrane and lyophilized once more. The pale yellow solid was dissolved in water (200 µL) and was applied on a Dowex sodium form column (Dowex® 50WX8 hydrogen form stored with an aqueous solution of 2N NaOH and washed until neutral pH with H₂O). The fractions containing the desired compound were combined, frozen and lyophilized to afford mono-(7-oxo-4-pyrazol-4-yl-1,6-diaza-bicyclo[3.2.1]oct-3-en-6-yl) ester sulfonate sodium salt (Example 1) (5.4 mg, 0.018 mmol, 49%) as a pale yellow solid.
MS *m*/*z* ([M-H]⁻) 285.
¹H NMR (400 MHz, D₂O): *δ* (ppm) 3.46 (d, *J=* 11.2 Hz, 1H), 3.71 (dd, *J=* 11.2/ 3.6 Hz, 1 H), 3.82 (dd, *J =* 18.6/ 3.6 Hz, 1 H), 3.99 (dd, *J =* 18.6/ 1.7 Hz, 1 H), 4.65-4.67 (m, 1 H), 5.91-5.94 (m, 1 H), 7.78-7.83 (bs, 2H).

### Example 2: synthesis of [4-(2-methylpyrazol-3-yl)-7-oxo-1,6-diazabicyclo[3.2.1]oct-3-en-6-yl] sulfate sodium salt

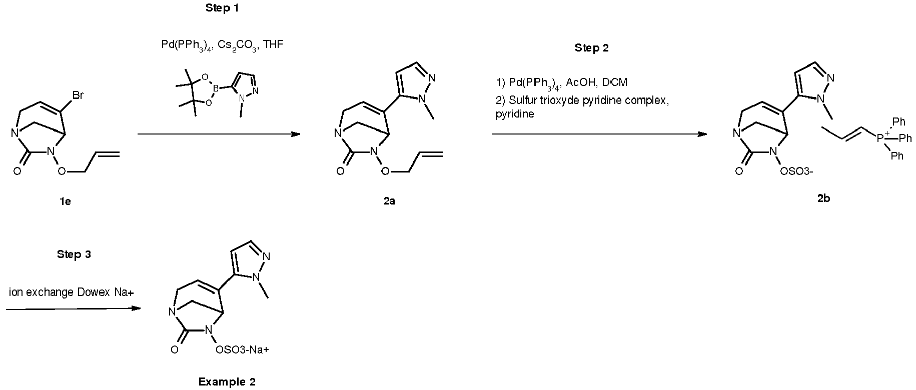

### Step 1: preparation of intermediate 6-allyloxy-4-(2-methylpyrazol-3-yl)-1,6-diazabicyclo[3.2.1]oct-3-en-7-one (2a)

In a Wheaton vial, 6-allyloxy-4-bromo-1,6-diazabicyclo[3.2.1]oct-3-en-7-one (1 e) (30 mg, 0.116 mmol), 1-methyl-1*H*-pyrazole-5-boronic acid pinacol ester (28.9 mg, 0.139 mmol) and anhydrous CsCO₃ (75.4 mg, 0.232 mmol) were dissolved in anhydrous THF (1.3 mL). The solution was degassed by bubbling argon for 10 min and Pd(PPh₃)₄ (4.0 mg, 0.003 mmol) was added. The reaction mixture was heated at 60 °C for 90 min. Water (1 mL) was added and the mixture was extracted with EtOAc (2 x 1 mL). The organic layer was dried over sodium sulfate, filtered and concentrated under *vacuum* to afford a crude material which was purified by preparative TLC (toluene/acetone: 8/2) to give the desired product 6-allyloxy-4-(2-methylpyrazol-3-yl)-1,6-diazabicyclo[3.2.1]oct-3-en-7-one (2a) (8.8 mg, 0.034 mmol, 29%).
MS *m*/*z* ([M+H]⁺) 261.
¹H NMR (300 MHz, CDCl₃): *δ* (ppm) 3.23 (m, 1 H), 3.58-3.63 (m, 1 H), 3.83-4.05 (m, 3H), 3.86 (s, 3H), 4.28-4.44 (m, 2H), 5.23-5.31 (m, 2H), 5.74-5.77 (m, 1 H), 5.82-5.96 (m, 1 H), 6.17 (d, *J* = 2.0 Hz, 1 H), 7.43 (d, *J* = 2.0 Hz, 1H).

### Step 2: preparation of intermediate [4-(2-methylpyrazol-3-yl)-7-oxo-1,6-diazabicyclo[3.2.1]oct-3-en-6-yl] sulfate triphenyl-(propenyl)-phosphonium salt (2b)

Using the procedure described in example 1 (step 6), the intermediate 6-allyloxy-4-(2-methylpyrazol-3-yl)-1,6-diazabicyclo[3.2.1]oct-3-en-7-one (2a) (52 mg, 0.198 mmol) is converted into [4-(2-methylpyrazol-3-yl)-7-oxo-1,6-diazabicyclo[3.2.1]oct-3-en-6-yl] sulfate triphenyl-(propenyl)-phosphonium salt (2b) (56.7 mg) as an amorphous solid after purification by flash chromatography on silica gel (DCM/acetone 80/20 to 0/100).
MS *m*/*z* ([M+H]⁺) 301.
MS *m*/*z* ([M-H]⁻) 299.
MS *m*/*z* ([M+H]⁺) 303 (triphenyl-propenyl-phosphonium).

### Step 3: preparation of [4-(2-methylpyrazol-3-yl)-7-oxo-1,6-diazabicyclo[3.2.1]oct-3-en-6-yl] sulfate sodium salt (Example 2)

Using the procedure described in example 1 (step 7), [4-(2-methylpyrazol-3-yl)-7-oxo-1,6-diazabicyclo[3.2.1]oct-3-en-6-yl] sulfate triphenyl-(propenyl)-phosphonium salt (2b) (56.7 mg) is converted after ion exchange (Dowex sodium form column) into [4-(2-methylpyrazol-3-yl)-7-oxo-1,6-diazabicyclo[3.2.1]oct-3-en-6-yl] sulfate sodium salt (Example 2) (34.6 mg, 0.107 mmol, 54% over 2 steps) as a white amorphous solid after lyophilization.
MS *m*/*z* ([M+H]⁺) 301.
MS *m*/*z* ([M-H]⁻) 299.
¹H NMR (300 MHz, D₂O): *δ* (ppm) 3.53-357 (m, 1 H), 3.73-3.95 (m, 5H), 4.07-4.14 (m, 1 H), 4.53-4.54 (m, 1 H), 6.00 (bs, 1 H), 6.41 (d, *J=* 2.2 Hz, 1 H), 7.52 (d, *J=* 2.2 Hz, 1H).

### Example 3: synthesis of [4-(oxazol-5-yl)-7-oxo-1,6-diazabicyclo[3.2.1]oct-3-en-6-yl] sulfate sodium salt

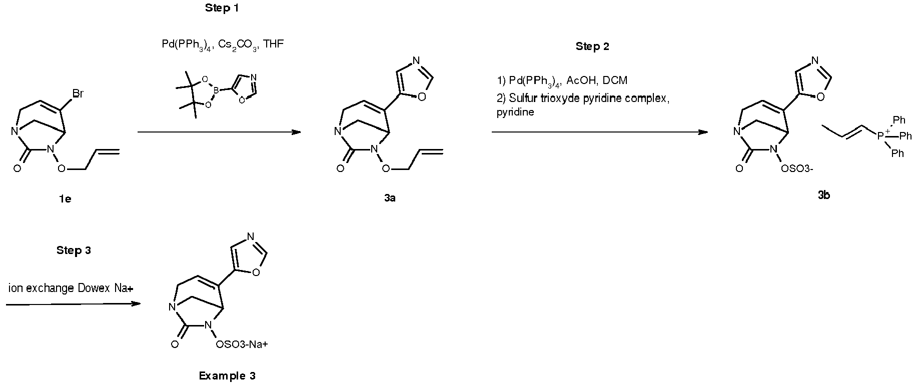

### Step 1: preparation of intermediate 6-allyloxy-4-(oxazol-5-yl)-1,6-diazabicyclo[3.2.1]oct-3-en-7-one (3a)

Using the procedure described in example 2 (step 1), the intermediate 6-allyloxy-4-bromo-1,6-diazabicyclo[3.2.1]oct-3-en-7-one (1 e) (30 mg, 0.116 mmol) is converted into 6-allyloxy-4-(oxazol-5-yl)-1,6-diazabicyclo[3.2.1]oct-3-en-7-one (3a) (2.4 mg, 0.010 mmol, 8.4%) using 5-(4,4,5,5-tetramethyl-1,3,2-dioxaborolan-2-yl)oxazole (27.1 mg, 0.139 mmol) and after purification by preparative TLC (toluene/acetone: 8/2).
MS *m*/*z* ([M+H]⁺) 248, ([M+Na]⁺) 270.
¹H NMR (300 MHz, CDCl₃): *δ* (ppm) 3.17 (d, *J =* 10.9 Hz, 1 H), 3.60 (dd, *J =* 10.9/3.0 Hz, 1 H), 3.84-4.00 (m, 2H), 4.17-4.18 (m, 1 H), 4.35-4.46 (m, 2H), 5.29-5.36 (m, 2H), 5.94-6.07 (m, 2H), 7.01 (s, 1 H), 7.81 (s, 1H).

### Step 2: preparation of intermediate [4-(oxazol-5-yl)-7-oxo-1,6-diazabicyclo[3.2.1]oct-3-en-6-yl] sulfate triphenyl-(propenyl)-phosphonium salt (3b)

Using the procedure described in example 1 (step 6), the intermediate 6-allyloxy-4-(oxazol-5-yl)-1,6-diazabicyclo[3.2.1]oct-3-en-7-one (3a) (47 mg, 0.190 mmol) is converted into [4-(oxazol-5-yl)-7-oxo-1,6-diazabicyclo[3.2.1]oct-3-en-6-yl] sulfate triphenyl-(propenyl)-phosphonium salt (3b) (60.6 mg) as an amorphous solid after purification by flash chromatography on silica gel (DCM/acetone 80/20 to 10/90).
MS *m*/*z* ([M+H]⁺) 288.
MS *m*/*z* ([M-H]⁻) 286.
MS *m*/*z* ([M+H]⁺) 303 (triphenyl-propenyl-phosphonium).

### Step 3 : preparation of [4-(oxazol-5-yl)-7-oxo-1,6-diazabicyclo[3.2.1]oct-3-en-6-yl] sulfate sodium salt (Example 3)

Using the procedure described in example 1 (step 7), [4-(oxazol-5-yl)-7-oxo-1,6-diazabicyclo[3.2.1]oct-3-en-6-yl] sulfate triphenyl-(propenyl)-phosphonium salt (3b) (60.6 mg) is converted after ion exchange (Dowex sodium form column) into [4-(oxazol-5-yl)-7-oxo-1,6-diazabicyclo[3.2.1]oct-3-en-6-yl] sulfate sodium salt (Example 3) (25.2 mg, 0.081 mmol, 43% over 2 steps) as a white amorphous solid after lyophilization.
MS *m*/*z* ([M+H]⁺) 288
MS *m*/*z* ([M-H]⁻) 286.
¹H NMR (300 MHz, D₂O): δ (ppm) 3.49 (dd, *J* = 11.4/0.7 Hz, 1H), 3.75 (ddd, *J* = 11.4/3.1/0.7 Hz, 1 H), 3.87-4.12 (m, 2H), 4.73-4.74 (m, 1 H), 6.22-6.24 (m, 1 H), 7.22 (s, 1H), 8.12 (s, 1H).

### Example 4: synthesis of [4-(oxazol-2-yl)-7-oxo-1,6-diazabicyclo[3.2.1]oct-3-en-6-yl] sulfate sodium salt

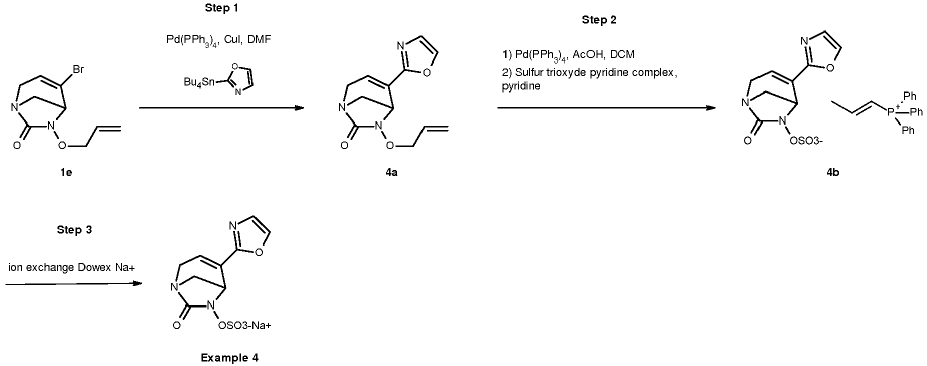

### Step 1: preparation of intermediate 6-allyloxy-4-(oxazol-2-yl)-1,6-diazabicyclo[3.2.1]oct-3-en-7-one (4a).

In a Wheaton vial, 6-allyloxy-4-bromo-1,6-diazabicyclo[3.2.1]oct-3-en-7-one (1 e) (30 mg, 0.116 mmol), 2-(tri-n-butylstannyl)oxazole (53.9 mg, 0.0.151 mmol) and anhydrous copper (I) iodide (22.0 mg, 0.116 mmol) were dissolved in anhydrous DMF (1.2 mL). The solution was degassed by bubbling argon for 5 min and Pd(PPh₃)₄ (13.4 mg, 0.012 mmol) was added. The reaction was stirred for 2 h at 80 °C. EtOAc (1 mL) was added, followed by a saturated solution of potassium fluoride (1 mL). The mixture was stirred at rt for 1 h. The organic layer was separated, washed with brine (2 mL), dried over sodium sulfate, filtered, and concentrated under *vacuum* to afford a crude material which was purified by preparative TLC (toluene/acetone: 7/3) to give the desired 6-allyloxy-4-(oxazol-2-yl)-1,6-diazabicyclo[3.2.1]oct-3-en-7-one (4a) (8.4 mg, 0.034 mmol, 29%).
MS *m*/*z* ([M+H]⁺) 248, ([M+Na]⁺) 270.
¹H NMR (300 MHz, CDCl₃): *δ* (ppm) 3.14 (d, *J =* 11.0 Hz, 1 H), 3.60 (dd, *J =* 11.0/3.0 Hz, 1 H), 3.83-4.03 (m, 2H), 4.36-4.46 (m, 2H), 4.75-4.76 (m, 1 H), 5.24-5.35 (m, 2H), 5.95-6.05 (m, 1 H), 6.50-6.51 (m, 1 H), 7.14 (d, *J=* 0.8 Hz, 1 H), 7.60 (bs, 1H).

### Step 2: preparation of intermediate [4-(oxazol-2-yl)-7-oxo-1,6-diazabicyclo[3.2.1]oct-3-en-6-yl] sulfate triphenyl-(propenyl)-phosphonium salt (4b)

Using the procedure described in example 1 (step 6), the intermediate 6-allyloxy-4-(oxazol-2-yl)-1,6-diazabicyclo[3.2.1]oct-3-en-7-one (4a) (71 mg, 0.287 mmol) is converted into [4-(oxazol-2-yl)-7-oxo-1,6-diazabicyclo[3.2.1]oct-3-en-6-yl] sulfate triphenyl-(propenyl)-phosphonium salt (4b) as an amorphous solid after purification by flash chromatography on silica gel (DCM/acetone from 80/20 to 10/90).
MS *m*/*z* ([M+H]⁺) 288.
MS *m*/*z* ([M-H]⁻) 286.
MS *m*/*z* ([M+H]⁺) 303 (triphenyl-propenyl-phosphonium).

### Step 3 : preparation of [4-(oxazol-2-yl)-7-oxo-1,6-diazabicyclo[3.2.1]oct-3-en-6-yl] sulfate sodium salt (Example 4)

Using the procedure described in example 1 (step 7), [4-(oxazol-2-yl)-7-oxo-1,6-diazabicyclo[3.2.1]oct-3-en-6-yl] sulfate triphenyl-(propenyl)-phosphonium salt (4b) is converted after ion exchange (Dowex sodium form column) into [4-(oxazol-2-yl)-7-oxo-1,6-diazabicyclo[3.2.1]oct-3-en-6-yl] sulfate sodium salt (Example 4) (24.4 mg, 0.079 mmol, 27% over 2 steps) as a white amorphous solid after lyophilization.
MS *m*/*z* ([M+H]⁺) 288.
MS *m*/*z* ([M-H]⁻) 286.
¹H NMR (300 MHz, D₂O): *δ* (ppm) 3.47 (dd, *J* = 11.4/0.7 Hz 1 H), 3.79 (ddd, *J* = 11.4/3.7/0.7 Hz 1H), 3.92-4.16 (m, 2H), 5.01-5.02 (m, 1H), 6.69-6.71 (m, 1H), 7.23 (d, *J=* 0.9 Hz, 1 H), 7.86 (d, *J* = 0.9 Hz, 1 H).

### Example 5: synthesis of (7-oxo-4-isoxazol-4-yl-1,6-diaza-bicyclo[3.2.1]oct-3-en-6-yl) sulfate sodium salt

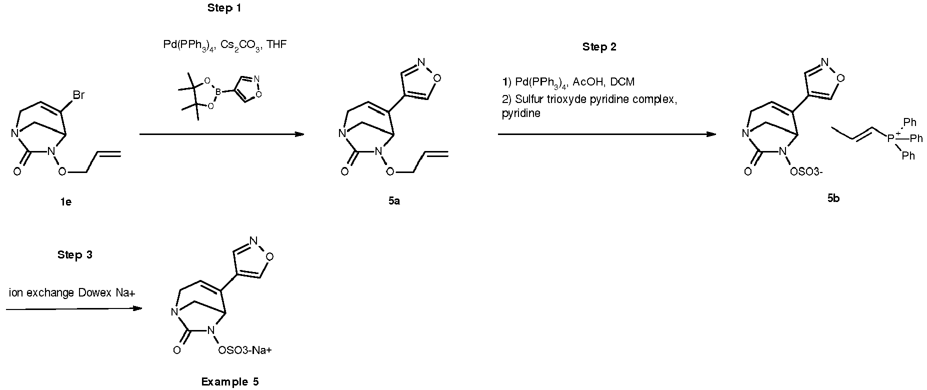

### Step 1: preparation of intermediate 6-allyloxy-4-isoxazol-4-yl-1,6-diazabicyclo[3.2.1]oct-3-en-7-one (5a)

In a Wheaton vial, 6-allyloxy-4-bromo-1,6-diazabicyclo[3.2.1]oct-3-en-7-one (1 e) (30 mg, 0.116 mmol), 4-(4,4,5,5-tetramethyl-1,3,2-dioxaborolan-2-yl)isoxazole (27.1 mg, 0.139 mmol) and anhydrous CsCO₃ (75.4 mg, 0.232 mmol) were dissolved in anhydrous THF (1.3 mL). The solution was degassed by bubbling argon for 10 min and Pd(PPh₃)₄ (4.0 mg, 0.003 mmol) was added. The reaction was stirred for 45 min at rt. Water (1 mL) was added and the mixture was extracted with EtOAc (2 x 1 mL). The organic layer was dried over sodium sulfate, filtered, and concentrated under *vacuum* to afford a crude material which was purified by preparative TLC (toluene/acetone: 8/2) to give the desired product 6-allyloxy-4-isoxazol-4-yl-1,6-diazabicyclo[3.2.1]oct-3-en-7-one (5a) (3.0 mg, 0.012 mmol, 10%).
MS *m*/*z* ([M+H]⁺) 248.
¹H NMR (400 MHz, CDCl₃): *δ* (ppm) 3.23 (dd, *J* = 10.9/0.4 Hz, 1H), 3.64 (ddd, *J* = 10.9/3.4/0.4 Hz, 1 H), 3.87 (dd, *J* = 18.8/2.1 Hz, 1 H), 3.99 (dd, *J* = 18.8/3.4 Hz, 1 H), 4.04-4.08 (m, 1 H), 4.39-4.57 (m, 2H), 5.34-5.48 (m, 2H), 5.85-5.90 (m, 1 H), 5.96-6.13 (m, 1 H), 8.36 (s, 1H), 8.42 (s, 1H).

### Step 2: preparation of intermediate (7-oxo-4-isoxazol-4-yl-1,6-diaza-bicyclo[3.2.1]oct-3-en-6-yl) sulfate triphenyl-(propenyl)-phosphonium salt (5b)

Using the procedure described in example 1 (step 6), the intermediate 6-allyloxy-4-isoxazol-4-yl-1,6-diazabicyclo[3.2.1]oct-3-en-7-one (5a) (35 mg, 0.142 mmol) was converted into (7-oxo-4-isoxazol-4-yl-1,6-diaza-bicyclo[3.2.1]oct-3-en-6-yl) ester sulfonate triphenyl-(propenyl)-phosphonium salt (5b) (57 mg) as an amorphous solid after purification by flash chromatography on silica gel (DCM/acetone 100/0 to 0/100).
MS *m*/*z* ([M+H]⁺) 288.
MS *m*/*z* ([M-H]⁻) 286.
MS *m*/*z* ([M+H]⁺) 303 (triphenyl-propenyl-phosphonium).

### Step 3 : preparation of (7-oxo-4-isoxazol-4-yl-1,6-diaza-bicyclo[3.2.1]oct-3-en-6-yl) sulfate sodium salt (Example 5)

Using the procedure described in example 1 (step 7), (7-oxo-4-isoxazol-4-yl-1,6-diaza-bicyclo[3.2.1]oct-3-en-6-yl) ester sulfonate triphenyl-(propenyl)-phosphonium salt (5b) (57 mg) was converted after ion exchange (Dowex sodium form column) into (7-oxo-4-isoxazol-4-yl-1,6-diaza-bicyclo[3.2.1]oct-3-en-6-yl) ester sulfonate sodium salt (Example 5) (19.4 mg, 0.062 mmol, 44% over 2 steps) as a white amorphous solid after lyophilization.
MS *m*/*z* ([M+H]⁺) 288.
MS *m*/*z* ([M-H]⁻) 286.
¹H NMR (400 MHz, D₂O): *δ* (ppm) 3.50 (d, *J* = 11.4 Hz, 1 H), 3.74 (dd, *J* = 11.4/3.0 Hz, 1 H), 3.87 (dd, *J* = 18.8/3.5 Hz, 1 H), 4.04 (dd, *J* = 18.8/2.0 Hz, 1 H), 4.65-4.69 (m, 1 H), 6.06-6.11 (m, 1 H), 8.65 (s, 1 H), 8.75 (s, 1H).

### Example 6: synthesis of difluoro-(4-isoxazol-4-yl-7-oxo-1,6-diaza-bicyclo[3.2.1]oct-3-en-6-yloxy)-acetate lithium salt

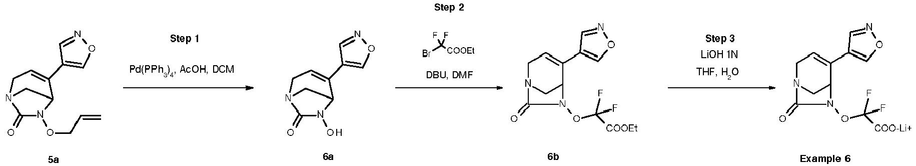

### Step 1: preparation of intermediate 6-hydroxy-4-isoxazol-4-yl-1,6-diazabicyclo[3.2.1]oct-3-en-7-one (6a)

To a solution of 6-allyloxy-4-isoxazol-4-yl-1,6-diazabicyclo[3.2.1]oct-3-en-7-one (5a) (400 mg, 1.62 mmol) and glacial AcOH (185 µL, 3.24 mmol) in anhydrous DCM (16 mL) was added in one portion Pd(PPh₃)₄ (935 mg, 0.81 mmol) at rt. After stirring 20 min, the mixture was evaporated under nitrogen flux. The oily residue was purified by chromatography on silica gel (DCM/Acetone 7/3) to afford 6-hydroxy-4-isoxazol-4-yl-1,6-diazabicyclo[3.2.1]oct-3-en-7-one (6a) (275 mg, 1.33 mmol, 82%).
MS *m*/*z* ([M+H]⁺) 208.
¹H NMR (300 MHz, CDCl₃): *δ* (ppm) 3.22 (d, *J* = 11.3 Hz, 1H), 3.63 (dd, *J* = 3.2/11.3 Hz, 1 H), 3.84 (dd, *J* = 2.2/18.8 Hz, 1 H), 3.93 (dd, *J* = 3.2/18.8 Hz, 1 H), 4.07 (dd, *J =* 1.1/2.5 Hz, 1 H), 5.84-5.86 (m, 1 H), 8.35 (s, 1 H), 8.47 (s, 1H).

### Step 2: preparation of intermediate ethyl 2,2-difluoro-2-[(4-isoxazol-4-yl-7-oxo-1,6-diazabicyclo[3.2.1]oct-3-en-6-yl)oxylacetate (6b)

6-hydroxy-4-isoxazol-4-yl-1,6-diazabicyclo[3.2.1]oct-3-en-7-one (6a) (227 mg, 1.09 mmol) was solubilized in DMF (12 mL) at -20 °C with DBU (179 µL, 1.20 mmol) and ethyl 2-bromo-2,2-difluoro-acetate (702 µL, 5.48 mmol). After stirring 1 h, the reaction mixture was evaporated under nitrogen flux. The residue was purified by chromatography on silica gel (DCM/Et₂O 9/1) to provide ethyl 2,2-difluoro-2-[(4-isoxazol-4-yl-7-oxo-1,6-diazabicyclo[3.2.1]oct-3-en-6-yl)oxy]acetate (6b) which was triturated with MTBE (214 mg, 0.65 mmol, 59%).
MS *m*/*z* ([M+H]⁺) 330.
¹H NMR (300 MHz, CDCl₃): *δ* (ppm) 1.34 (t, *J* = 7.2 Hz, 3H), 3.29 (d, *J* = 11.4 Hz, 1 H), 3.70 (dd, *J* = 2.7/11.4 Hz, 1 H), 3.93 (dd, *J* = 2.1/18.8 Hz, 1 H), 4.05 (dd, *J* = 3.4/18.8 Hz, 1 H), 4.28 (d, *J =* 2.7 Hz, 1 H), 4.36 (q, *J =* 7.2 Hz, 2H), 5.91-5.95 (m, 1 H), 8.37 (s, 1 H), 8.52 (s, 1H).

### Step 3: preparation of difluoro-(4-isoxazol-4-yl-7-oxo-1,6-diaza-bicyclo[3.2.1]oct-3-en-6-yloxy)-acetate lithium salt (Example 6)

Ethyl 2,2-difluoro-2-[(4-isoxazol-4-yl-7-oxo-1,6-diazabicyclo[3.2.1]oct-3-en-6-yl)oxy]acetate (6b) (188 mg, 0.57 mmol) was solubilized in THF (3.3 mL) and water (2.1 mL) at 0 °C. A solution of LiOH 1 N (730 µL, 0.73 mmol) was then dropped. The mixture was stirred for 1 h at 0 °C. The reaction mixture was acidified with HCl 0.5N (330 µL, 0.16 mmol) and concentrated to remove THF. The resulting aqueous layer was frozen and lyophilized. The resulting salt was solubilized with isopropanol and filtrated on a silica gel cake. The filtrate was concentrated and the residue was triturated with MTBE to provide difluoro-(4-isoxazol-4-yl-7-oxo-1,6-diaza-bicyclo[3.2.1]oct-3-en-6-yloxy)-acetate lithium salt (Example 6) (131 mg, 0.43 mmol, 76%) as a white solid.
MS *m*/*z* ([M+H]⁺) 208.
¹H NMR (400 MHz, DMSO-*d₆*): *δ* (ppm) 3.29 (d, *J =* 11.2 Hz, 1 H), 3.42 (dd, *J* = 2.8/11.3 Hz, 1 H), 3.73-3.81 (m, 1 H), 3.92 (dd, *J* = 1.7/18.6 Hz, 1 H), 4.44 (d, *J =* 2.3 Hz, 1 H), 6.03-6.07 (m, 1 H), 8.96 (s, 1 H), 9.28 (s, 1H).

### Example 7: synthesis of [4-(4-pyridyl)-7-oxo-1,6-diazabicyclo[3.2.1]oct-3-en-6-yl] sulfate sodium salt

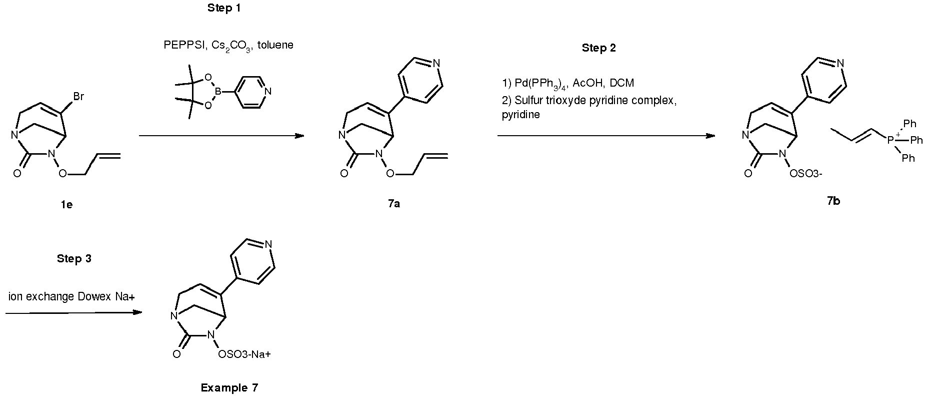

### Step 1: preparation of intermediate 6-allyloxy-4-(4-pyridyl)-1,6-diazabicyclo[3.2.1]oct-3-en-7-one (7a)

In a Wheaton vial, 6-allyloxy-4-bromo-1,6-diazabicyclo[3.2.1]oct-3-en-7-one (1 e) (30 mg, 0.116 mmol), 4-pyridineboronic acid pinacol ester (28.5 mg, 0.139 mmol) and CsCO₃ (75.4 mg, 0.232 mmol) were dissolved in anhydrous toluene (1.2 mL). The solution was degassed by bubbling argon for 10 min and PEPPSI catalyst (3.9 mg, 0.004 mmol) was added. The reaction was stirred for 2 h at 100 °C under microwave. Water (1 mL) was added and the mixture was extracted with EtOAc (2 x 1 mL). The organic layer was dried over sodium sulfate, filtered and concentrated under reduced pressure to afford a crude material which was purified by preparative TLC (toluene/acetone 55/45) to give the desired 6-allyloxy-4-(4-pyridyl)-1,6-diazabicyclo[3.2.1]oct-3-en-7-one (7a) (6.0 mg, 0.023 mmol, 20%).
MS *m*/*z* ([M+H]⁺) 258.
¹H NMR (300 MHz, CDCl₃): *δ* (ppm) 3.21 (d, *J =* 10.9 Hz, 1 H), 3.69 (dd, *J* = 10.9/3.0 Hz, 1 H), 3.90 (dd, *J* = 19.0/2.0 Hz, 1 H), 4.03 (dd, *J* = 19.0/2.0 Hz, 1 H), 4.29 (d, *J =* 2.3 Hz, 1 H), 4.43-4.58 (m, 2H), 5.34-5.36 (m, 1 H), 5.37-5.42 (m, 1 H), 6.00-6.10 (m, 1 H), 6.13-6.17 (m, 1 H), 7.26-7.30 (m, 2H), 8.59-8.63 (m, 2H).

### Step 2: preparation of intermediate [4-(4-pyridyl)-7-oxo-1,6-diazabicyclo[3.2.1]oct-3-en-6-yl] sulfate triphenyl-(propenyl)-phosphonium salt (7b)

Using the procedure described in example 1 (step 6), the intermediate 6-allyloxy-4-(4-pyridyl)-1,6-diazabicyclo[3.2.1]oct-3-en-7-one (7a) (50 mg, 0.194 mmol) was converted into [4-(4-pyridyl)-7-oxo-1,6-diazabicyclo[3.2.1]oct-3-en-6-yl] sulfate triphenyl-(propenyl)-phosphonium salt (7b) as an amorphous solid after purification by flash chromatography on silica gel (DCM/acetone 50/50 to acetone/pyridine 96/4).
MS *m*/*z* ([M+H]⁺) 298.
MS *m*/*z* ([M-H]⁻) 296.
MS *m*/*z* ([M+H]⁺) 303 (triphenyl-propenyl-phosphonium).

### Step 3: preparation of [4-(4-pyridyl)-7-oxo-1,6-diazabicyclo[3.2.1]oct-3-en-6-yl] sulfate sodium salt (Example 7)

Using the procedure described in example 1 (step 7), [4-(4-pyridyl)-7-oxo-1,6-diazabicyclo[3.2.1]oct-3-en-6-yl] sulfate triphenyl-(propenyl)-phosphonium salt (7b) was converted after ion exchange (Dowex sodium form column) into [4-(4-pyridyl)-7-oxo-1,6-diazabicyclo[3.2.1]oct-3-en-6-yl] sulfate sodium salt (Example 7) (3.9 mg, 0.013 mmol, 7 % over 2 steps) as a white amorphous solid after lyophilization.
MS *m*/*z* ([M+H]⁺) 298.
MS *m*/*z* ([M-H]⁻) 296.
¹H NMR (400 MHz, D₂O): *δ* (ppm) 3.47 (d, *J* = 11.3 Hz, 1 H), 3.78 (dd, *J* = 11.3/3.2 Hz, 1 H), 3.92 (dd, *J* = 19.2/3.5 Hz, 1 H), 4.07 (dd, *J* = 19.2/2.2 Hz, 1 H), 4.82-4.85 (m, 1 H), 6.39-6.42 (m, 1 H), 7.47-7.50 (m, 2H), 8.47-8.51 (m, 2H).

### Example 8: synthesis of [4-(N-methyl-6-oxo-3-pyridyl)-7-oxo-1,6-diazabicyclo[3.2.1]oct-3-en-6-yl] sulfate sodium salt

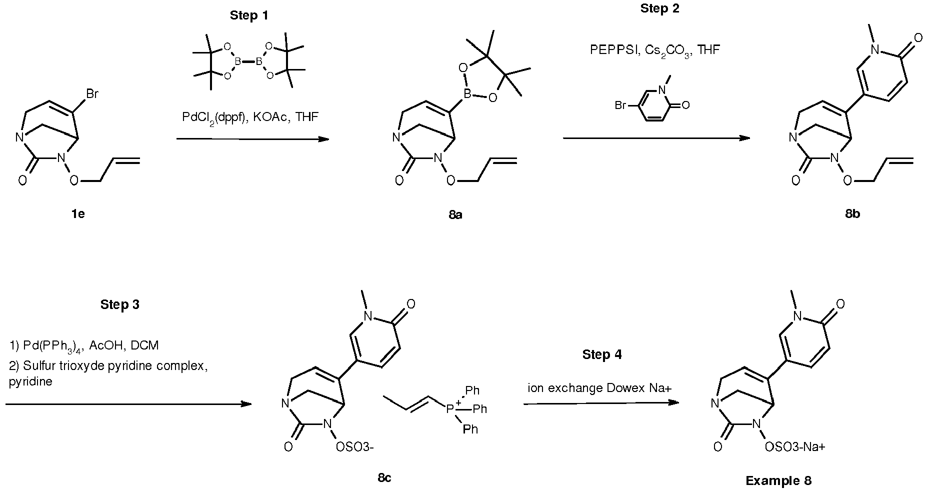

### Step 1: preparation of intermediate (6-allyloxy-4-(4,4,5,5-tetramethyl-1,3,2-dioxaborolan-2-yl)-1,6-diazabicyclo[3.2.1]oct-3-en-7-one (8a)

In a Wheaton vial, 6-allyloxy-4-bromo-1,6-diazabicyclo[3.2.1]oct-3-en-7-one (1 e) (10.0 mg, 0.039 mmol), KOAc (11.4 mg, 0.116 mmol) and bis(pinacolato)diboron (11.8 mg, 0.046 mmol) were dissolved in anhydrous THF (0.5 mL). The solution was degassed by bubbling argon for 10 min and Pd(dppf)Cl₂ (1.6 mg, 0.002 mmol) was added. The reaction was stirred for 60 min at 80 °C under microwaves. Water (1 mL) was added and the mixture was extracted with EtOAc (2 x 1 mL). The organic layer was dried over sodium sulfate, filtered, and concentrated under *vacuum* to afford a crude material which was purified by chromatography on silica gel (cyclohexane/EtOAc 8/2) to give the desired product (6-allyloxy-4-(4,4,5,5-tetramethyl-1,3,2-dioxaborolan-2-yl)-1,6-diazabicyclo[3.2.1] oct-3-en-7-one (8a) (2.2 mg, 0.007 mmol, 19%).
MS *m*/*z* ([M+H]⁺) 307.
¹H NMR (300 MHz, CDCl₃): *δ* (ppm) 1.24 (s, 6H), 1.25 (s, 6H), 3.04 (d, *J =* 10.7 Hz, 1 H), 3.45 (dd, *J* = 10.7/2.9 Hz, 1H), 3.74 (dd, *J* = 19.2/1.9 Hz, 1H), 3.86 (dd, *J* = 19.2/3.1 Hz, 1 H), 4.10 (d, *J* = 2.9 Hz, 1 H), 4.34-4.48 (m, 2H), 5.25 (dq, *J* = 10.4/1.2 Hz, 1 H), 5.35 (dq, *J* = 17.2/1.5 Hz, 1 H), 5.96-6.08 (m, 1 H), 6.41-6.45 (m, 1 H).

### Step 2: preparation of intermediate 6-allyloxy-4-(N-methyl-6-oxo-3-pyridyl)-1,6-diazabicyclo[3.2.1]oct-3-en-7-one (8b)

In a Wheaton vial, (6-allyloxy-4-(4,4,5,5-tetramethyl-1,3,2-dioxaborolan-2-yl)-1,6-diazabicyclo[3.2.1]oct-3-en-7-one (8a) (30 mg, 0.098 mmol), 5-bromo-*N*-methylpyridin-2(1*H*)-one (22.1 mg, 0.118 mmol) and CsCO₃ (63.8 mg, 0.196 mmol) were dissolved in anhydrous THF (0.7 mL). The solution was degassed by bubbling argon for 10 min and PEPPSI catalyst (3.3 mg, 0.005 mmol) was added. The reaction was stirred for 2h at 80 °C under microwaves. The mixture was filtered and concentrated under reduced pressure to afford a crude material which was purified by preparative TLC (DCM/EtOAc 60/40) to give the 6-allyloxy-4-(*N*-methyl-6-oxo-3-pyridyl)-1,6-diazabicyclo[3.2.1]oct-3-en-7-one (8b) (4.0 mg, 0.014 mmol, 14%).
MS *m*/*z* ([M+H]⁺) 288.
¹H NMR (300 MHz, CDCl₃): *δ* (ppm) 3.09 (d, *J =* 10.8 Hz, 1 H), 3.47 (s, 3H), 3.55 (dd, *J* = 10.8/3.0 Hz, 1H), 3.74 (dd, *J* = 18.7/2.1 Hz, 1H), 3.87 (dd, *J* = 18.7/3.4 Hz, 1H), 3.98-4.01 (m, 1 H), 4.33-4.51 (m, 2H), 5.25-5.36 (m, 2H), 5.60-5.64 (m, 1 H), 5.89-6.05 (m, 1 H), 6.47-6.52 (m, 1 H), 7.20-7.35 (m, 2H).

### Step 3: preparation of intermediate [4-(N-methyl-6-oxo-3-pyridyl)-7-oxo-1,6-diazabicyclo[3.2.1]oct-3-en-6-yl] sulfate triphenyl-(propenyl)-phosphonium salt (8c)

Using the procedure described in example 1 (step 6), the intermediate 6-allyloxy-4-(*N-*methyl-6-oxo-3-pyridyl)-1,6-diazabicyclo[3.2.1]oct-3-en-7-one (8b) (33 mg, 0.115 mmol) was converted into [4-(*N*-methyl-6-oxo-3-pyridyl)-7-oxo-1,6-diazabicyclo[3.2.1]oct-3-en-6-yl] sulfate triphenyl-(propenyl)-phosphonium salt (8c) as an amorphous solid after purification by flash chromatography on silica gel (DCM/acetone 50/50 to 0/100).
MS *m*/*z* ([M+H]⁺) 328.
MS *m*/*z* ([M-H]⁻) 326.
MS *m*/*z* ([M+H]⁺) 303 (triphenyl-propenyl-phosphonium).

### Step 4: preparation of [4-(N-methyl-6-oxo-3-pyridyl)-7-oxo-1,6-diazabicyclo[3.2.1]oct-3-en-6-yl] sulfate sodium salt (Example 8)

Using the procedure described in example 1 (step 7), [4-(*N*-methyl-6-oxo-3-pyridyl)-7-oxo-1,6-diazabicyclo[3.2.1]oct-3-en-6-yl] sulfate triphenyl-(propenyl)-phosphonium salt (8c) was converted after ion exchange (Dowex sodium form column) into [4-(*N*-methyl-6-oxo-3-pyridyl)-7-oxo-1,6-diazabicyclo[3.2.1]oct-3-en-6-yl] sulfate sodium salt (Example 8) (10.8 mg, 0.031 mmol, 27% over 2 steps) as a white amorphous solid after lyophilization.
MS *m*/*z* ([M+H]⁺) 328.
MS *m*/*z* ([M-H]⁻) 326.
¹H NMR (400 MHz, D₂O): *δ* (ppm) 3.46 (d, *J =* 11.3 Hz, 1 H), 3.58 (s, 3H), 3.71-3.76 (m, 1 H), 3.85 (dd, *J* = 18.9/3.6 Hz, 1 H), 4.02 (dd, *J* = 18.8/2.1 Hz, 1 H), 4.68-4.71 (m, 1 H), 5.96-6.00 (m, 1 H), 6.61-6.65 (m, 1 H), 7.74-7.78 (m, 2H).

### Example 9: synthesis of [4-(3-pyridyl)-7-oxo-1,6-diazabicyclo[3.2.1]oct-3-en-6-yl] sulfate sodium salt

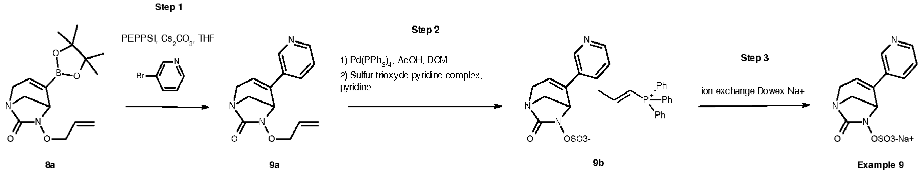

### Step 1: preparation of intermediate 6-allyloxy-4-(3-pyridyl)-1,6-diazabicyclo[3.2.1]oct-3-en-7-one (9a)

In a vial, (6-allyloxy-4-(4,4,5,5-tetramethyl-1,3,2-dioxaborolan-2-yl)-1,6-diazabicyclo[3.2.1] oct-3-en-7-one (8a) (200 mg, 0.653 mmol), 3-bromopyridine (155 mg, 0.980 mmol), dry CsCO₃ (424 mg, 1.306 mmol) were dissolved in anhydrous THF (4 mL). The solution was degassed under argon for 5 min and PEPPSI catalyst (89 mg, 0.130 mmol) was added. The reaction was stirred at 80 °C for 1 h under microwaves. The mixture was filtered and concentrated under reduced pressure to afford a crude material which was purified by chromatography on silica gel (DCM/acetone 100/0 to 50/50) to give the desired product 6-allyloxy-4-(3-pyridyl)-1,6-diazabicyclo[3.2.1]oct-3-en-7-one (9a) (68 mg, 0.264 mmol, 27%) as a gum.
MS *m*/*z* ([M+H]⁺) 258.
¹H NMR (300 MHz, CDCl₃): *δ* (ppm) 3.21 (d, *J =* 10.8 Hz, 1 H), 3.65 (dd, *J* = 10.8/3.0 Hz, 1 H), 3.86 (dd, *J* = 18.8/2.1 Hz, 1 H), 3.99 (dd, *J* = 18.8/3.4 Hz, 1 H), 4.22-4.24 (m, 1 H), 4.41-4.54 (m, 2H), 5.29-5.33 (m, 1 H), 5.33-5.40 (m, 1 H), 5.94-5.97 (m, 1 H), 5.98-6.07 (m, 1 H), 7.26-7.30 (m, 1 H), 7.66-7.70 (m, 1 H), 8.52 (dd, *J* = 4.8/1.6 Hz, 1 H), 8.61-8.64 (m, 1 H).

### Step 2: preparation of intermediate [4-(3-pyridyl)-7-oxo-1,6-diazabicyclo[3.2.1]oct-3-en-6-yl] sulfate triphenyl-(propenyl)-phosphonium salt (9b)

Using the procedure described in example 1 (step 6), the intermediate 6-allyloxy-4-(3-pyridyl)-1,6-diazabicyclo[3.2.1]oct-3-en-7-one (9a) (102 mg, 0.397 mmol) was converted into [4-(3-pyridyl)-7-oxo-1,6-diazabicyclo[3.2.1]oct-3-en-6-yl] sulfate triphenyl-(propenyl)-phosphonium salt (9b) as an amorphous solid. Crude product was used in the next step without purification.
MS *m*/*z* ([M+H]⁺) 298.
MS *m*/*z* ([M-H]⁻) 296.
MS *m*/*z* ([M+H]⁺) 303 (triphenyl-propenyl-phosphonium).

### Step 3: preparation of [4-(3-pyridyl)-7-oxo-1,6-diazabicyclo[3.2.1]oct-3-en-6-yl] sulfate sodium salt (Example 9)

Using the procedure described in example 1 (step 7), [4-(3-pyridyl)-7-oxo-1,6-diazabicyclo[3.2.1]oct-3-en-6-yl] sulfate triphenyl-(propenyl)-phosphonium salt (9b) was converted after ion exchange (Dowex sodium form column) into [4-(3-pyridyl)-7-oxo-1,6-diazabicyclo[3.2.1]oct-3-en-6-yl] sulfate sodium salt. After lyophilization, the residue was purified by chromatography on C18 (water/ACN 99/1). After a passage on G10 column (water elution), [4-(3-pyridyl)-7-oxo-1,6-diazabicyclo[3.2.1]oct-3-en-6-yl] sulfate sodium salt (Example 9) (5.3 mg, 0.017 mmol, 5% over 2 steps) was recovered as a white amorphous solid after lyophilization.
MS *m*/*z* ([M+H]⁺) 298.
MS *m*/*z* ([M-H]⁻) 296.
¹H NMR (400 MHz, D₂O): *δ* (ppm) 3.49 (d, *J* = 11.3 Hz, 1 H), 3.76 (dd, *J* = 11.3/3.1 Hz, 1 H), 3.90 (dd, *J* = 18.9/3.4 Hz, 1 H), 4.05 (dd, *J* = 18.9/1.9 Hz, 1 H), 4.76-4.78 (m, 1 H), 6.16-6.19 (m, 1 H), 7.44 (dd, *J* = 8.0/4.9 Hz, 1 H), 7.86-7.91 (m, 1 H), 8.40-8.44 (m, 1 H), 8.56-8.59 (m, 1H).

### Example 10: synthesis of [4-(pyrimidin-5-yl)-7-oxo-1,6-diazabicyclo[3.2.1]oct-3-en-6-yl] sulfate sodium salt

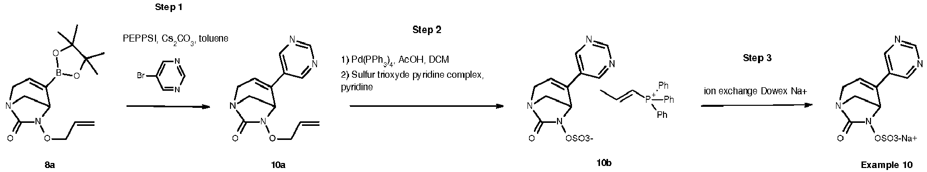

### Step 1: preparation of intermediate 6-allyloxy-4-(pyrimidin-5-yl)-1,6-diazabicyclo[3.2.1]oct-3-en-7-one (10a)

In a Wheaton vial, (6-allyloxy-4-(4,4,5,5-tetramethyl-1,3,2-dioxaborolan-2-yl)-1,6-diazabicyclo[3.2.1]oct-3-en-7-one (8a) (250 mg, 0.816 mmol), 5-bromopyrimidine (156 mg, 0.980 mmol), dry CsCO₃ (530 mg, 1.630 mmol) were dissolved in anhydrous toluene (5 mL). The solution was degassed under argon for 5 min and PEPPSI catalyst (28 mg, 0.041 mmol) was added. The reaction was stirred at 100 °C for 1.5 h under microwaves. The mixture was filtered and concentrated under reduced pressure to afford a crude material which was purified by chromatography on silica gel (DCM/acetone 100/0 to 50/50) to give the desired product 6-allyloxy-4-(pyrimidin-5-yl)-1,6-diazabicyclo[3.2.1]oct-3-en-7-one (10a) (55 mg, 0.213 mmol, 26%) as a gum.
MS *m*/*z* ([M+H]⁺) 259.
¹H NMR (300 MHz, CDCl₃): *δ* (ppm) 3.21 (d, *J =* 10.9 Hz, 1 H), 3.66 (dd, *J* = 10.9/3.0 Hz, 1 H), 3.86 (dd, *J* = 19.0/2.1 Hz, 1 H), 4.00 (dd, *J* = 19.0/3.4 Hz, 1 H), 4.18-4.21 (m, 1 H), 4.39-4.54 (m, 2H), 5.29-5.40 (m, 2H), 5.94-6.06 (m, 2H), 8.73 (s, 2H), 9.11 (s, 1 H).

### Step 2: preparation of intermediate [4-(pyrimidin-5-yl)-7-oxo-1,6-diazabicyclo[3.2.1]oct-3-en-6-yl] sulfate triphenyl-(propenyl)-phosphonium salt (10b)

Using the procedure described in example 1 (step 6), the intermediate 6-allyloxy-4-(pyrimidin-5-yl)-1,6-diazabicyclo[3.2.1]oct-3-en-7-one (10a) (102 mg, 0.397 mmol) was converted into [4-(pyrimidin-5-yl)-7-oxo-1,6-diazabicyclo[3.2.1]oct-3-en-6-yl] sulfate triphenyl-(propenyl)-phosphonium salt (10b) as an amorphous solid. Crude product was used in the next step without purification.
MS *m*/*z* ([M+H]⁺) 299.
MS *m*/*z* ([M-H]⁻) 297.
MS *m*/*z* ([M+H]⁺) 303 (triphenyl-propenyl-phosphonium).

### Step 3: preparation of [4-(pyrimidin-5-yl)-7-oxo-1,6-diazabicyclo[3.2.1]oct-3-en-6-yl] sulfate sodium salt (Example 10)

Using the procedure described in example 1 (step 7), [4-(pyrimidin-5-yl)-7-oxo-1,6-diazabicyclo[3.2.1]oct-3-en-6-yl] sulfate triphenyl-(propenyl)-phosphonium salt (10b) was converted after ion exchange (Dowex sodium form column) into [4-(pyrimidin-5-yl)-7-oxo-1,6-diazabicyclo[3.2.1]oct-3-en-6-yl] sulfate sodium salt. After lyophilization, the residue was purified by chromatography on C18 (water/ACN 99/1) to give [4-(pyrimidin-5-yl)-7-oxo-1,6-diazabicyclo[3.2.1]oct-3-en-6-yl] sulfate sodium salt (Example 10) (2.4 mg, 0.007 mmol, 4% over 2 steps) as a white amorphous solid after lyophilization.
MS *m*/*z* ([M+H]⁺) 299.
MS *m*/*z* ([M-H]⁻) 297.
¹H NMR (400 MHz, D₂O): *δ* (ppm) 3.53 (d, *J* = 11.4 Hz, 1 H), 3.81 (dd, *J* = 11.4/3.2 Hz, 1 H), 3.96 (dd, *J =* 19.2/3.5 Hz, 1 H), 4.10 (dd, *J =* 19.2/2.1 Hz, 1 H), 4.82-4.85 (m, 1 H), 6.34-6.37 (m, 1 H), 8.87 (s, 2H), 9.04 (s, 1 H).

### Example 11: synthesis of [4-(2-pyridyl)-7-oxo-1,6-diazabicyclo[3.2.1]oct-3-en-6-yl] sulfate sodium salt

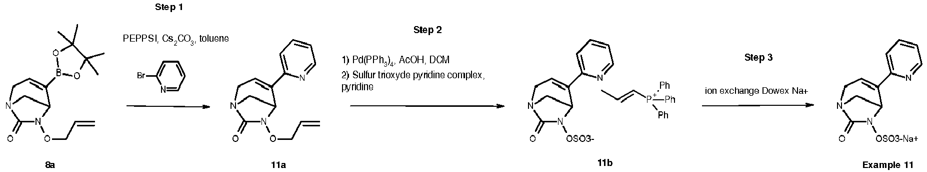

### Step 1: preparation of intermediate 6-allyloxy-4-(2-pyridyl)-1,6-diazabicyclo[3.2.1]oct-3-en-7-one (11 a)

Using the procedure described in example 10 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (50 mg, 0.163 mmol) is converted into 6-allyloxy-4-(2-pyridyl)-1,6-diazabicyclo[3.2.1]oct-3-en-7-one (8a) (19 mg, 0.074 mmol, 45%) using 2-bromopyridine (31 mg, 0.196 mmol), PEPPSI catalyst (22 mg, 0.032 mmol) and after purification by chromatography on silica gel (DCM/acetone 100/0 to 80/20).
MS *m*/*z* ([M+H]⁺) 258.
¹H NMR (300 MHz, CDCl₃): *δ* (ppm) 3.17 (d, *J =* 10.9 Hz, 1 H), 3.63 (dd, *J* = 10.9/3.1 Hz, 1 H), 3.87 (dd, *J* = 19.0/2.2 Hz, 1 H), 4.00 (dd, *J* = 19.0/3.4 Hz, 1 H), 4.31-4.45 (m, 2H), 4.98-5.02 (m, 1 H), 5.19-5.25 (m, 1 H), 5.28-5.30 (m, 1 H), 5.84-5.99 (m, 1 H), 6.30-6.34 (m, 1 H), 7.16 (ddd, *J* = 7.5/4.9 Hz, *J =* 1.0 Hz, 1 H), 7.43-7.48 (m, 1 H), 7.65 (dd, *J* = 7.7/1.8 Hz, 1 H), 8.54 (ddd, *J* = 4.9/1.8/1.0 Hz, 1H).

### Step 2: preparation of intermediate [4-(2-pyridyl)-7-oxo-1,6-diazabicyclo[3.2.1]oct-3-en-6-yl] sulfate triphenyl-(propenyl)-phosphonium salt (11b)

Using the procedure described in example 1 (step 6), the intermediate 6-allyloxy-4-(2-pyridyl)-1,6-diazabicyclo[3.2.1]oct-3-en-7-one (11 a) (100 mg, 0.389 mmol) was converted into [4-(2-pyridyl)-7-oxo-1,6-diazabicyclo[3.2.1]oct-3-en-6-yl] sulfate triphenyl-(propenyl)-phosphonium salt (11 b) as an amorphous solid which was used in the next step without purification.
MS *m*/*z* ([M+H]⁺) 298.
MS *m*/*z* ([M-H]⁻) 296.
MS *m*/*z* ([M+H]⁺) 303 (triphenyl-propenyl-phosphonium).

### Step 3: preparation of [4-(2-pyridvy)-7-oxo-1,6-diazabicyclo[3.2.1]oct-3-en-6-yl] sulfate sodium salt (Example 11)

Using the procedure described in example 1 (step 7), [4-(2-pyridyl)-7-oxo-1,6-diazabicyclo[3.2.1]oct-3-en-6-yl] sulfate triphenyl-(propenyl)-phosphonium salt (11b) was converted after ion exchange (Dowex sodium form column) into [4-(2-pyridyl)-7-oxo-1,6-diazabicyclo[3.2.1]oct-3-en-6-yl] sulfate sodium salt. After lyophilization, the residue was purified by chromatography on C18 (water/ACN 99/1) to give [4-(2-pyridyl)-7-oxo-1,6-diazabicyclo[3.2.1]oct-3-en-6-yl] sulfate sodium salt (Example 11) (1.2 mg, 0.004 mmol, 1 % over 2 steps) as a white amorphous solid after lyophilization.
MS *m*/*z* ([M+H]⁺) 298.
MS *m*/*z* ([M-H]⁻) 296.
¹H NMR (400 MHz, D₂O): *δ* (ppm) 3.49 (d, *J* = 11.3 Hz, 1 H), 3.80 (dd, *J* = 11.3/3.2 Hz, 1 H), 3.94 (dd, *J =* 19.1/3.5 Hz, 1 H), 4.10 (dd, *J =* 19.1/2.1 Hz, 1 H), 5.02-5.04 (m, 1 H), 6.48-6.51 (m, 1 H), 7.35-7.40 (m, 1 H), 7.58-7.63 (m, 1 H), 7.87-7.92 (m, 1 H), 8.48-8.51 (m, 1 H).

### Example 12: synthesis of [4-(pyrazin-2-yl)-7-oxo-1,6-diazabicyclo[3.2.1]oct-3-en-6-yl] sulfate sodium salt

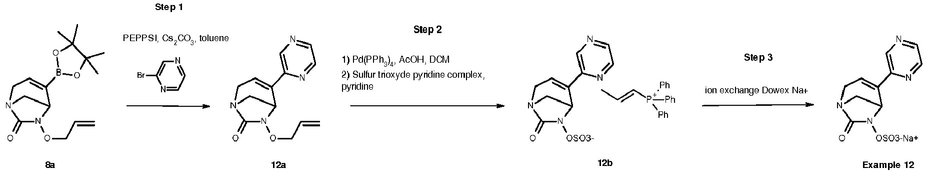

### Step 1: preparation of intermediate 6-allyloxy-4-(pyrazin-2-yl)-1,6-diazabicyclo[3.2.1]oct-3-en-7-one (12a)

Using the procedure described in example 10 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (250 mg, 0.817 mmol) is converted into 6-allyloxy-4-(pyrazin-2-yl)-1,6-diazabicyclo[3.2.1]oct-3-en-7-one (12a) (57 mg, 0.221 mmol, 27%) using 2-bromopyrazine (195 mg, 1.220 mmol), PEPPSI catalyst (111 mg, 0.163 mmol) and after purification by chromatography on silica gel (DCM/acetone 100/0 to 80/20).
MS *m*/*z* ([M+H]⁺) 259.
¹H NMR (300 MHz, CDCl₃): *δ* (ppm) 3.18 (d, *J =* 10.9 Hz, 1 H), 3.67 (dd, *J* = 10.9/2.9 Hz, 1 H), 3.91 (dd, *J* = 19.2/2.2 Hz, 1 H), 4.04 (dd, *J* = 19.2/3.4 Hz, 1 H), 4.32-4.45 (m, 2H), 4.91-4.94 (m, 1 H), 5.21-5.32 (m, 2H), 5.86-6.00 (m, 1 H), 6.48-6.52 (m, 1 H), 8.44 (d, *J* = 2.5 Hz, 1 H), 8.50 (dd, *J* = 2.5/1.5 Hz, 1 H), 8.77 (d, *J=* 1.5 Hz, 1H).

### Step 2: preparation of intermediate [4-(pyrazin-2-yl)-7-oxo-1,6-diazabicyclo[3.2.1]oct-3-en-6-yl] sulfate triphenyl-(propenyl)-phosphonium salt (12b)

Using the procedure described in example 1 (step 6), the intermediate 6-allyloxy-4-(pyrazin-2-yl)-1,6-diazabicyclo[3.2.1]oct-3-en-7-one (12a) (57 mg, 0.220 mmol) was converted into [4-(pyrazin-2-yl)-7-oxo-1,6-diazabicyclo[3.2.1]oct-3-en-6-yl] sulfate triphenyl-(propenyl)-phosphonium salt (12b) as an amorphous solid which was used in the next step without purification.
MS *m*/*z* ([M+H]⁺) 298.
MS *m*/*z* ([M-H]⁻) 296.
MS *m*/*z* ([M+H]⁺) 303 (triphenyl-propenyl-phosphonium).

### Step 3: preparation of [4-(pyrazin-2-yl)-7-oxo-1,6-diazabicyclo[3.2.1]oct-3-en-6-yl] sulfate sodium salt (Example 12)

Using the procedure described in example 1 (step 7), [4-(pyrazin-2-yl)-7-oxo-1,6-diazabicyclo[3.2.1]oct-3-en-6-yl] sulfate triphenyl-(propenyl)-phosphonium salt (12b) was converted after ion exchange (Dowex sodium form column) into [4-(pyrazin-2-yl)-7-oxo-1,6-diazabicyclo[3.2.1]oct-3-en-6-yl] sulfate sodium salt. After lyophilization, the residue was purified by chromatography on C18 (water/ACN 99/1) to give [4-(pyrazin-2-yl)-7-oxo-1,6-diazabicyclo[3.2.1]oct-3-en-6-yl] sulfate sodium salt (Example 12) (1.2 mg, 0.004 mmol, 2% over 2 steps) as a white amorphous solid after lyophilization.
MS *m*/*z* ([M+H]⁺) 298.
MS *m*/*z* ([M-H]⁻) 296.
¹H NMR (400 MHz, D₂O): *δ* (ppm) 3.50 (d, *J* = 11.4 Hz, 1 H), 3.82 (dd, *J* = 11.4/3.1 Hz, 1 H), 3.98 (dd, *J* = 19.4/3.5 Hz, 1 H), 4.13 (dd, *J* = 19.4/2.0 Hz, 1 H), 5.12-5.15 (m, 1 H), 6.65-6.68 (m, 1 H), 8.49-8.52 (m, 1 H), 8.58-8.61 (m, 1 H), 8.79-8.81 (m, 1H).

### Example 13: synthesis of [4-(N-methyl-2-oxo-4-pyridyl)-7-oxo-1,6-diazabicyclo[3.2.1]oct-3-en-6-yl] sulfate sodium salt

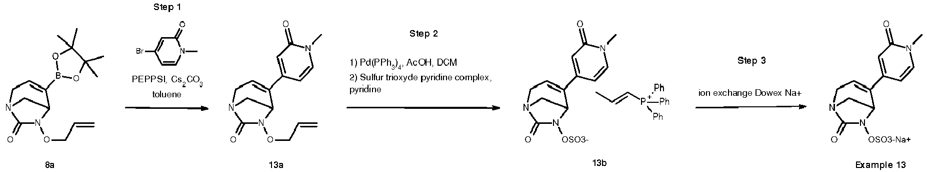

### Step 1: preparation of intermediate 6-allyloxy-4-(N-methyl-2-oxo-4-pyridyl)-1,6-diazabicyclo[3.2.1]oct-3-en-7-one (13a)

Using the procedure described in example 10 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (250 mg, 0.817 mmol) is converted into 6-allyloxy-4-(*N*-methyl-2-oxo-4-pyridyl)-1,6-diazabicyclo[3.2.1]oct-3-en-7-one (13a) (100 mg, 0.348 mmol, 43%) using 4-bromo-*N-*methyl-pyridin-2-one (230 mg, 1.220 mmol), PEPPSI catalyst (111 mg, 0.163 mmol) and after purification by chromatography on silica gel (DCM/acetone 100/0 to 50/50).
MS *m*/*z* ([M+H]⁺) 288.
¹H NMR (300 MHz, CDCl₃): *δ* (ppm) 3.12 (d, *J* = 10.9 Hz, 1 H), 3.50 (s, 3H), 3.61 (dd, *J* = 10.9/3.0 Hz, 1 H), 3.83 (dd, *J* = 19.1/2.0 Hz, 1 H), 3.95 (dd, *J* = 19.1/3.4 Hz, 1 H), 4.19-4.22 (m, 1 H), 4.37-4.48 (m, 2H), 5.28-5.39 (m, 2H), 5.96-6.05 (m, 1 H), 6.05-6.08 (m, 1 H), 6.19 (dd, *J* = 7.1/2.0 Hz, 1 H), 6.49 (d, *J* = 1.9 Hz, 1 H), 7.22 (d, *J* = 7.1 Hz, 1H).

### Step 2: preparation of intermediate [4-(N-methyl-2-oxo-4-pyridyl)-7-oxo-1,6-diazabicyclo[3.2.1]oct-3-en-6-yl] sulfate triphenyl-(propenyl)-phosphonium salt (13b)

Using the procedure described in example 1 (step 6), the intermediate 6-allyloxy-4-(*N-*methyl-2-oxo-4-pyridyl)-1,6-diazabicyclo[3.2.1]oct-3-en-7-one (13a) (57 mg, 0.220 mmol) was converted into [4-(*N*-methyl-2-oxo-4-pyridyl)-7-oxo-1,6-diazabicyclo[3.2.1]oct-3-en-6-yl] sulfate triphenyl-(propenyl)-phosphonium salt (13b) as an amorphous solid after purification by flash chromatography on silica gel (DCM/acetone 50/50 to 0/100).
MS *m*/*z* ([M+H]⁺) 328.
MS *m*/*z* ([M-H]⁻) 326.
MS *m*/*z* ([M+H]⁺) 303 (triphenyl-propenyl-phosphonium).

### Step 3: preparation of [4-(1-methyl-2-oxo-4-pyridyl)-7-oxo-1,6-diazabicyclo[3.2.1]oct-3-en-6-yl] sulfate sodium salt (Example 13)

Using the procedure described in example 1 (step 7), [4-(*N*-methyl-2-oxo-4-pyridyl)-7-oxo-1,6-diazabicyclo[3.2.1]oct-3-en-6-yl] sulfate triphenyl-(propenyl)-phosphonium salt (13b) was converted after ion exchange (Dowex sodium form column) into [4-(*N*-methyl-2-oxo-4-pyridyl)-7-oxo-1,6-diazabicyclo[3.2.1]oct-3-en-6-yl] sulfate sodium salt (Example 13) (18.2 mg, 0.052 mmol, 15% over 2 steps) as a white amorphous solid after lyophilization.
MS *m*/*z* ([M+H]⁺) 328.
MS *m*/*z* ([M-H]⁻) 326.
¹H NMR (400 MHz, D₂O): *δ* (ppm) 3.40 (d, *J =* 11.3 Hz, 1 H), 3.49 (s, 3H), 3.75 (dd, *J* = 10.8/3.1 Hz, 1H), 3.87 (dd, *J* = 19.4/3.4 Hz, 1H), 4.05 (dd, *J* = 19.4/2.0 Hz, 1H), 4.69-4.72 (m, 1 H), 6.30-6.33 (m, 1 H), 6.51-6.55 (m, 1 H), 6.55-6.58 (m, 1 H), 7.52 (d, *J* = 7.1 Hz, 1H).

### Example 14: synthesis of [4-(4-methyl-5-oxo-pyrazin-2-yl)-7-oxo-1,6-diazabicyclo[3.2.1]oct-3-en-6-yl] sulfate sodium salt

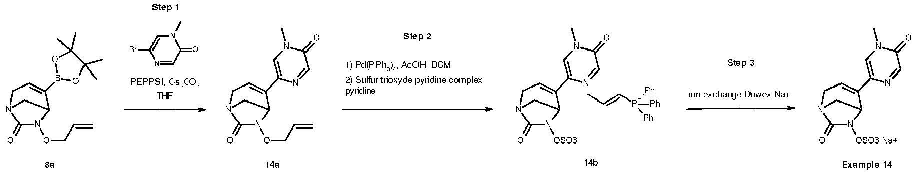

### Step 1: preparation of intermediate 6-alyvloxy-4-(4-methyl-5-oxo-prazin-2-yl)-1,6-diazabicyclo[3.2.1]oct-3-en-7-one (14a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(4-methyl-5-oxo-pyrazin-2-yl)-1,6-diazabicyclo[3.2.1]oct-3-en-7-one (14a) (65 mg, 0.225 mmol, 35%) using 5-bromo-1-methyl-pyrazin-2-one (150 mg, 0.790 mmol), PEPPSI catalyst (89 mg, 0.130 mmol) and after purification by chromatography on silica gel (DCM/acetone 100/0 to 50/50).
MS *m*/*z* ([M+H]⁺) 289.
¹H NMR (300 MHz, CDCl₃): *δ* (ppm) 3.15 (d, *J* = 10.9 Hz, 1 H), 3.52 (s, 3H), 3.58-3.64 (m, 1 H), 3.82-3.97 (m, 2H), 4.32-4.47 (m, 2H), 4.51 (d, *J =* 2.7 Hz, 1 H), 5.26-5.36 (m, 2H), 5.90-6.01 (m, 1 H), 6.04-6.08 (m, 1H), 7.24 (bs, 1H), 8.10 (bs, 1H).

### Step 2: preparation of intermediate [4-(4-methyl-5-oxo-pyrazin-2-yl)-7-oxo-1,6-diazabicyclo[3.2.1]oct-3-en-6-yl] sulfate triphenyl-(propenyl)-phosphonium salt (14b)

Using the procedure described in example 1 (step 6), the intermediate 6-allyloxy-4-(4-methyl-5-oxo-pyrazin-2-yl)-1,6-diazabicyclo[3.2.1]oct-3-en-7-one (14a) (65 mg, 0.225 mmol) was converted into [4-(4-methyl-5-oxo-pyrazin-2-yl)-7-oxo-1,6-diazabicyclo[3.2.1]oct-3-en-6-yl] sulfate triphenyl-(propenyl)-phosphonium salt (14b) as an amorphous solid after purification by flash chromatography on silica gel (DCM/acetone 50/50 to 0/100).
MS *m*/*z* ([M+H]⁺) 329.
MS *m*/*z* ([M-H]⁻) 327.
MS *m*/*z* ([M+H]⁺) 303 (triphenyl-propenyl-phosphonium).

### Step 3: preparation of [4-(4-methyl-5-oxo-pyrazin-2-yl)-7-oxo-1,6-diazabicyclo[3.2.1]oct-3-en-6-yl] sulfate sodium salt (Example 14)

Using the procedure described in example 1 (step 7), [4-(4-methyl-5-oxo-pyrazin-2-yl)-7-oxo-1,6-diazabicyclo[3.2.1]oct-3-en-6-yl] sulfate triphenyl-(propenyl)-phosphonium salt (14b) was converted after ion exchange (Dowex sodium form column) into [4-(4-methyl-5-oxo-pyrazin-2-yl)-7-oxo-1,6-diazabicyclo[3.2.1]oct-3-en-6-yl] sulfate sodium salt (Example 14) (20 mg, 0.057 mmol, 26% over 2 steps) as a white amorphous solid after lyophilization.
MS *m*/*z* ([M+H]⁺) 329.
MS *m*/*z* ([M-H]⁻) 327.
¹H NMR (400 MHz, DMSO): *δ* (ppm) 3.21 (d, *J* = 11.0 Hz, 1 H), 3.39 (s, 3H), 3.45 (dd, *J* = 11.0/3.0 Hz, 1 H), 3.68 (dd, *J* = 18.7/3.6 Hz, 1 H), 3.80 (dd, *J* = 18.7/1.9 Hz, 1 H), 4.62 (d, *J* = 1.9 Hz, 1H ), 6.18-6.22 (m, 1H), 7.94 (bs, 1H), 8.01 (d, *J* = 0.9 Hz, 1H).

### Example 15: synthesis of [4-(N-methyl-2-oxo-pyrimidin-5-yl)-7-oxo-1,6-diazabicyclo[3.2.1]oct-3-en-6-yl] sulfate sodium salt

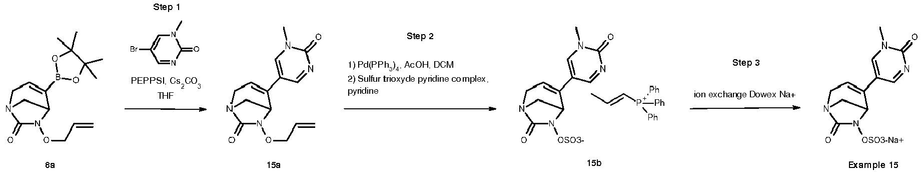

### Step 1: preparation of intermediate 6-allyloxy-4-(N-methyl-2-oxo-pyrimidin-5-yl)-1,6-diazabicyclo[3.2.1]oct-3-en-7-one (15a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(*N*-methyl-2-oxo-pyrimidin-5-yl)-1,6-diazabicyclo[3.2.1]oct-3-en-7-one (15a) (55 mg, 0.190 mmol, 30%) using 5-bromo-*N-*methyl-pyrimidin-2-one (150 mg, 0.790 mmol), PEPPSI catalyst (89 mg, 0.130 mmol) and after purification by chromatography on silica gel (DCM/acetone 100/0 to 50/50).
MS *m*/*z* ([M+H]⁺) 289.
¹H NMR (300 MHz, CDCl₃): *δ* (ppm) 3.18 (d, *J =* 10.6 Hz, 1 H), 3.56 (s, 3H), 3.62 (dd, *J* = 10.9/2.9 Hz, 1 H), 3.83 (dd, *J* = 18.8/2.0 Hz, 1 H), 3.95 (dd, *J* = 18.8/3.3 Hz, 1 H), 4.00-4.04 (m, 1 H), 4.39-4.57 (m, 2H), 5.33-5.43 (m, 2H), 5.77-5.81 (m, 1 H), 5.95-6.10 (m, 1 H), 7.68 (d, *J=* 3.3 Hz, 1 H), 8.63 (d, *J=* 3.3 Hz, 1H).

### Step 2: preparation of intermediate [4-(N-methyl-2-oxo-pyrimidin-5-yl)-7-oxo-1,6-diazabicyclo[3.2.1]oct-3-en-6-yl] sulfate triphenyl-(propenyl)-phosphonium salt (15b)

Using the procedure described in example 1 (step 6), the intermediate 6-allyloxy-4-(*N-*methyl-2-oxo-pyrimidin-5-yl)-1,6-diazabicyclo[3.2.1]oct-3-en-7-one (15a) (55 mg, 0.190 mmol) was converted into [4-(*N*-methyl-2-oxo-pyrimidin-5-yl)-7-oxo-1,6-diazabicyclo[3.2.1]oct-3-en-6-yl] sulfate triphenyl-(propenyl)-phosphonium salt (15b) as an amorphous solid after purification by flash chromatography on silica gel (DCM/acetone 50/50 to 0/100).
MS *m*/*z* ([M+H]⁺) 329.
MS *m*/*z* ([M-H]⁻) 327.
MS *m*/*z* ([M+H]⁺) 303 (triphenyl-propenyl-phosphonium).

### Step 3: preparation of [4-(N-methyl-2-oxo-pyrimidin-5-yl)-7-oxo-1,6-diazabicyclo[3.2.1]oct-3-en-6-yl] sulfate sodium salt (Example 15)

Using the procedure described in example 1 (step 7), [4-(*N*-methyl-2-oxo-pyrimidin-5-yl)-7-oxo-1,6-diazabicyclo[3.2.1]oct-3-en-6-yl] sulfate triphenyl-(propenyl)-phosphonium salt (15b) was converted after ion exchange (Dowex sodium form column) into [4-(*N*-methyl-2-oxo-pyrimidin-5-yl)-7-oxo-1,6-diazabicyclo[3.2.1]oct-3-en-6-yl] sulfate sodium salt (Example 15) (5.9 mg, 0.017 mmol, 9% over 2 steps) as a white amorphous solid after lyophilization.
MS *m*/*z* ([M+H]⁺) 329.
MS *m*/*z* ([M-H]⁻) 327.
¹H NMR (400 MHz, D₂O): *δ* (ppm) 3.49 (d, *J =* 11.3 Hz, 1 H), 3.61 (s, 3H), 3.75 (dd, *J* = 11.4/3.1 Hz, 1 H), 3.89 (dd, *J* = 18.9/3.5 Hz, 1H), 4.04 (dd, *J* = 18.9/2.0 Hz, 1 H), 4.66-4.69 (m, 1 H), 6.07-6.11 (m, 1 H), 8.22 (d, *J=* 3.2 Hz, 1 H), 8.74 (d, *J=* 3.2 Hz, 1H).

### Example 16: synthesis of [4-(N-methyl-2-oxo-3-pyridyl)-7-oxo-1,6-diazabicyclo[3.2.1]oct-3-en-6-yl] sulfate sodium salt

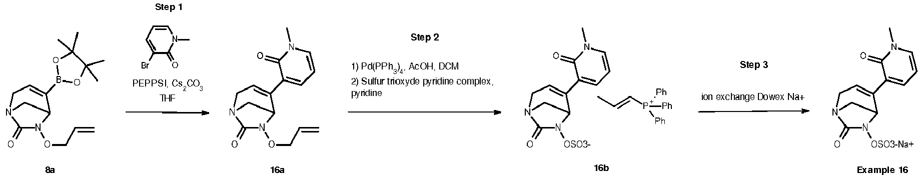

### Step 1: preparation of intermediate 6-allyloxy-4-(N-methyl-2-oxo-3-pyridyl)-1,6-diazabicyclo[3.2.1]oct-3-en-7-one (16a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(*N*-methyl-2-oxo-3-pyridyl)-1,6-diazabicyclo[3.2.1]oct-3-en-7-one (16a) (117 mg, 0.407 mmol, 63%) using 3-bromo-*N-*methyl-pyridin-2-one (149 mg, 0.790 mmol), PEPPSI catalyst (89 mg, 0.130 mmol) and after purification by chromatography on silica gel (DCM/acetone 100/0 to 50/50).
MS *m*/*z* ([M+H]⁺) 288.
¹H NMR (300 MHz, CDCl₃): *δ* (ppm) 3.32 (d, *J* = 10.8 Hz, 1 H), 3.55 (s, 3H), 3.55-3.59 (m, 1 H), 3.87 (d, *J =* 2.6 Hz, 2H), 4.25-4.40 (m, 2H), 4.51 (d, *J =* 2.6 Hz, 1 H), 5.18-5.28 (m, 2H), 5.82-5.95 (m, 2H), 6.18 (t, *J=* 6.8 Hz, 1 H), 7.27 (dd, *J* = 6.8/2.0 Hz, 1 H), 7.37 (dd, *J* = 6.9/2.0 Hz, 1H).

### Step 2: preparation of intermediate [4-(N-methyl-2-oxo-3-pyridyl)-7-oxo-1,6-diazabicyclo[3.2.1]oct-3-en-6-yl] sulfate triphenyl-(propenyl)-phosphonium salt (16b)

Using the procedure described in example 1 (step 6), the intermediate 6-allyloxy-4-(*N-*methyl-2-oxo-3-pyridyl)-1,6-diazabicyclo[3.2.1]oct-3-en-7-one (16a) (117 mg, 0.407 mmol) was converted into [4-(*N*-methyl-2-oxo-3-pyridyl)-7-oxo-1,6-diazabicyclo[3.2.1]oct-3-en-6-yl] sulfate triphenyl-(propenyl)-phosphonium salt (16b) as an amorphous solid after purification by flash chromatography on silica gel (DCM/acetone 50/50 to 0/100).
MS *m*/*z* ([M+H]⁺) 328.
MS *m*/*z* ([M-H]⁻) 326.
MS *m*/*z* ([M+H]⁺) 303 (triphenyl-propenyl-phosphonium).

### Step 3: preparation of [4-(N-methyl-2-oxo-3-pyridyl)-7-oxo-1,6-diazabicyclo[3.2.1]oct-3-en-6-yl] sulfate sodium salt (Example 16)

Using the procedure described in example 1 (step 7), [4-(*N*-methyl-2-oxo-3-pyridyl)-7-oxo-1,6-diazabicyclo[3.2.1]oct-3-en-6-yl] sulfate triphenyl-(propenyl)-phosphonium salt (16b) was converted after ion exchange (Dowex sodium form column) into [4-(*N*-methyl-2-oxo-3-pyridyl)-7-oxo-1,6-diazabicyclo[3.2.1]oct-3-en-6-yl] sulfate sodium salt (Example 16) (24 mg, 0.068 mmol, 17% over 2 steps) as a white amorphous solid after lyophilization.
MS *m*/*z* ([M+H]⁺) 328.
MS *m*/*z* ([M-H]⁻) 326.
¹H NMR (400 MHz, D₂O): *δ* (ppm) 3.54 (d, *J =* 11.2 Hz, 1 H), 3.56 (s, 3H), 3.74 (dd, *J* = 11.2/3.2 Hz, 1 H), 3.84 (dd, *J* = 18.8/3.4 Hz, 1 H), 4.07 (dd, *J* = 18.8/2.0 Hz, 1 H), 4.74-4.76 (m, 1H), 6.15-6.18 (m, 1H), 6.50 (t, *J=* 6.9 Hz, 1H), 7.58-7.63 (m, 2H).

### Example 17: synthesis of [4-(1-methyl-6-oxo-pyridazin-3-yl)-7-oxo-1,6-diazabicyclo[3.2.1]oct-3-en-6-yl] sulfate sodium salt

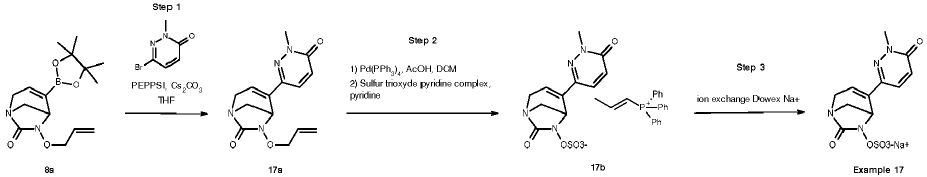

### Step 1: preparation of intermediate 6-allyloxy-4-(1-methyl-6-oxo-pyridazin-3-yl)-1,6-diazabicyclo[3.2.1]oct-3-en-7-one (17a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(1-methyl-6-oxo-pyridazin-3-yl)-1,6-diazabicyclo[3.2.1]oct-3-en-7-one (17a) (140 mg, 0.486 mmol, 75%) using 6-bromo-2-methyl-pyridazin-3-one (150 mg, 0.790 mmol), PEPPSI catalyst (89 mg, 0.130 mmol) and after purification by chromatography on silica gel (DCM/acetone 100/0 to 50/50).
MS *m*/*z* ([M+H]⁺) 289.
¹H NMR (400 MHz, CDCl₃): δ (ppm) 3.10 (d, *J =* 11.0 Hz, 1 H), 3.61 (dd, *J* = 11.0/3.0 Hz, 1 H), 3.78 (s, 3H), 3.85 (dd, *J* = 19.1/2.1 Hz, 1 H), 3.98 (dd, *J* = 19.1/3.4 Hz, 1 H), 4.33-4.45 (m, 2H), 4.90 (d, *J* = 2.4 Hz, 1 H), 5.24-5.34 (m, 2H), 5.89-6.00 (m, 1 H), 6.09-6.12 (m, 1 H), 6.91 (d, *J* = 9.7 Hz, 1 H), 7.43 (d, *J=* 9.7 Hz, 1H).

### Step 2: preparation of intermediate [4-(1-methyl-6-oxo-pyridazin-3-yl)-7-oxo-1,6-diazabicyclo[3.2.1]oct-3-en-6-yl] sulfate triphenyl-(propenyl)-phosphonium salt (17b)

Using the procedure described in example 1 (step 6), the intermediate 6-allyloxy-4-(1-methyl-6-oxo-pyridazin-3-yl)-1,6-diazabicyclo[3.2.1]oct-3-en-7-one (17a) (140 mg, 0.486 mmol) was converted into [4-(1-methyl-6-oxo-pyridazin-3-yl)-7-oxo-1,6-diazabicyclo[3.2.1]oct-3-en-6-yl] sulfate triphenyl-(propenyl)-phosphonium salt (17b) as an amorphous solid after purification by flash chromatography on silica gel (DCM/acetone 50/50 to 0/100).
MS *m*/*z* ([M+H]⁺) 329.
MS *m*/*z* ([M-H]⁻) 327.
MS *m*/*z* ([M+H]⁺) 303 (triphenyl-propenyl-phosphonium).

### Step 3: preparation of [4-(1-methyl-6-oxo-pyridazin-3-yl)-7-oxo-1,6-diazabicyclo[3.2.1]oct-3-en-6-yl] sulfate sodium salt (Example 17)

Using the procedure described in example 1 (step 7), [4-(1-methyl-6-oxo-pyridazin-3-yl)-7-oxo-1,6-diazabicyclo[3.2.1]oct-3-en-6-yl] sulfate triphenyl-(propenyl)-phosphonium salt (17b) was converted after ion exchange (Dowex sodium form column) into [4-(1-methyl-6-oxo-pyridazin-3-yl)-7-oxo-1,6-diazabicyclo[3.2.1]oct-3-en-6-yl] sulfate sodium salt (Example 16) (36 mg, 0.103 mmol, 22% over 2 steps) as a white amorphous solid after lyophilization.
MS *m*/*z* ([M+H]⁺) 329.
MS *m*/*z* ([M-H]⁻) 327.
¹H NMR (400 MHz, D₂O): *δ* (ppm) 3.44 (d, *J* = 11.3 Hz, 1 H), 3.78 (dd, *J* = 11.4/3.1 Hz, 1 H), 3.81 (s, 3H), 3.93 (dd, *J* = 19.2/3.5 Hz, 1 H), 4.09 (dd, *J* = 19.2/2.1 Hz, 1 H), 5.20 (d, *J* = 2.2 Hz, 1 H), 6.41-6.44 (m, 1H), 7.05 (d, *J* = 9.6 Hz, 1 H), 7.79 (d, *J* = 9.6 Hz, 1H).

### Example 18: synthesis of [4-(N-methyl-6-oxo-2-pyridyl)-7-oxo-1,6-diazabicyclo[3.2.1]oct-3-en-6-yl] sulfate sodium salt

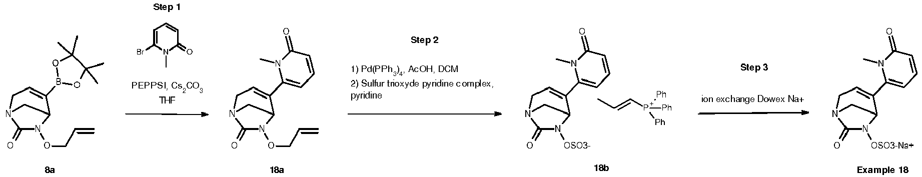

### Step 1: preparation of intermediate 6-allyloxy-4-(N-methyl-6-oxo-2-pyridyl)-1,6-diazabicyclo[3.2.1]oct-3-en-7-one (18a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(*N*-methyl-6-oxo-2-pyridyl)-1,6-diazabicyclo[3.2.1]oct-3-en-7-one (18a) (51 mg, 0.177 mmol, 28%) using 6-bromo-*N-*methyl-pyridin-2-one (150 mg, 0.790 mmol), PEPPSI catalyst (89 mg, 0.130 mmol) and after purification by chromatography on silica gel (DCM/acetone 100/0 to 50/50).
MS *m*/*z* ([M+H]⁺) 288.
¹H NMR (400 MHz, CDCl₃): *δ* (ppm) 3.23 (d, *J =* 10.9 Hz, 1 H), 3.41 (s, 3H), 3.65 (dd, *J* = 10.9/2.9 Hz, 1 H), 3.86 (dd, *J* = 18.9/1.9 Hz, 1 H), 3.94-4.01 (m, 2H), 4.23-4.40 (m, 2H), 5.20-5.28 (m, 2H), 5.76-5.87 (m, 2H), 6.15 (dd, *J* = 6.8/1.3 Hz, 1 H), 6.54 (dd, *J* = 9.1/1.3 Hz, 1 H), 7.28 (dd, *J* = 9.1/6.8 Hz, 1H).

### Step 2: preparation of intermediate [4-(N-methyl-6-oxo-2-pyridyl)-7-oxo-1,6-diazabicyclo[3.2.1]oct-3-en-6-yl] sulfate triphenyl-(propenyl)-phosphonium salt (18b)

Using the procedure described in example 1 (step 6), the intermediate 6-allyloxy-4-(*N-*methyl-6-oxo-2-pyridyl)-1,6-diazabicyclo[3.2.1]oct-3-en-7-one (18a) (51 mg, 0.177 mmol) was converted into [4-(*N-*methyl-6-oxo-2-pyridyl)-7-oxo-1,6-diazabicyclo[3.2.1]oct-3-en-6-yl] sulfate triphenyl-(propenyl)-phosphonium salt (18b) as an amorphous solid after purification by flash chromatography on silica gel (DCM/acetone 50/50 to 0/100).
MS *m*/*z* ([M+H]⁺) 328.
MS *m*/*z* ([M-H]⁻) 326.
MS *m*/*z* ([M+H]⁺) 303 (triphenyl-propenyl-phosphonium).

### Step 3: preparation of [4-(N-methyl-6-oxo-2-pyridyl)-7-oxo-1,6-diazabicyclo[3.2.1]oct-3-en-6-yl] sulfate sodium salt (Example 18)

Using the procedure described in example 1 (step 7), [4-(*N*-methyl-6-oxo-2-pyridyl)-7-oxo-1,6-diazabicyclo[3.2.1]oct-3-en-6-yl] sulfate triphenyl-(propenyl)-phosphonium salt (18b) was converted after ion exchange (Dowex sodium form column) into [4-(*N*-methyl-6-oxo-2-pyridyl)-7-oxo-1,6-diazabicyclo[3.2.1]oct-3-en-6-yl] sulfate sodium salt (Example 18) (7.5 mg, 0.021 mmol, 12% over 2 steps) as a white amorphous solid after lyophilization.
MS *m*/*z* ([M+H]⁺) 328.
MS *m*/*z* ([M-H]⁻) 326.
¹H NMR (400 MHz, D₂O): *δ* (ppm) 3.49 (s, 3H), 3.68 (d, *J* = 11.4 Hz, 1 H), 3.80 (dd, *J* = 11.4/2.9 Hz, 1 H), 3.94 (dd, *J* = 19.0/3.3 Hz, 1H), 4.11 (dd, *J* = 19.0/1.8 Hz, 1 H), 4.54 (d, *J* = 2.2 Hz, 1 H), 6.08-6.11 (m, 1 H), 6.56 (dd, *J* = 7.0/0.8 Hz, 1 H), 6.63 (dd, *J* = 9.0/0.8 Hz, 1 H), 7.63 (dd, *J* = 9.0/7.0 Hz, 1H).

### Example 19: synthesis of [4-(1-methyl-6-oxo-pyridazin-4-yl)-7-oxo-1,6-diazabicyclo[3.2.1]oct-3-en-6-yl] sulfate sodium salt

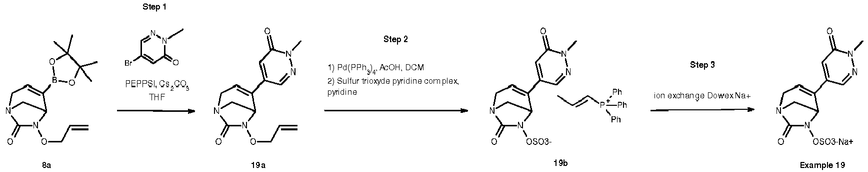

### Step 1: preparation of intermediate 6-allyloxy-4-(1-methyl-6-oxo-pyridazin-4-yl)-1,6-diazabicyclo[3.2.1]oct-3-en-7-one (19a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(1-methyl-6-oxo-pyridazin-4-yl)-1,6-diazabicyclo[3.2.1]oct-3-en-7-one (19a) (65 mg, 0.225 mmol, 35%) using 5-bromo-2-methyl-pyridazin-3-one (186 mg, 0.790 mmol), PEPPSI catalyst (89 mg, 0.130 mmol) and after purification by chromatography on silica gel (DCM/acetone 100/0 to 20/80).
MS *m*/*z* ([M+H]⁺) 289.
¹H NMR (400 MHz, CDCl₃): δ (ppm) 3.10 (d, *J =* 10.8 Hz, 1 H), 3.63 (dd, *J* = 10.8/3.2 Hz, 1 H), 3.75 (s, 3H), 3.86 (dd, *J* = 19.6/2.0 Hz, 1 H), 3.98 (dd, *J* = 19.2/3.2 Hz, 1 H), 4.15 (d, *J* = 2.0 Hz, 1 H), 4.38-4.48 (m, 2H), 5.30-5.38 (m, 2H), 5.95-6.05 (m, 1 H), 6.18 (s, 1 H), 6.78 (d, *J* = 2.0 Hz, 1 H), 7.80 (d, *J* = 2.0 Hz, 1H).

### Step 2: preparation of intermediate [4-(1-methyl-6-oxo-pyridazin-4-yl)-7-oxo-1,6-diazabicyclo[3.2.1]oct-3-en-6-yl] sulfate triphenyl-(propenyl)-phosphonium salt (19b)

Using the procedure described in example 1 (step 6), the intermediate 6-allyloxy-4-(1-methyl-6-oxo-pyridazin-4-yl)-1,6-diazabicyclo[3.2.1]oct-3-en-7-one (19a) (65 mg, 0.225 mmol) was converted into [4-(1-methyl-6-oxo-pyridazin-4-yl)-7-oxo-1,6-diazabicyclo[3.2.1]oct-3-en-6-yl] sulfate triphenyl-(propenyl)-phosphonium salt (19b) as an amorphous solid after purification by flash chromatography on silica gel (DCM/acetone 50/50 to 0/100).
MS *m*/*z* ([M+H]⁺) 329.
MS *m*/*z* ([M-H]⁻) 327.
MS *m*/*z* ([M+H]⁺) 303 (triphenyl-propenyl-phosphonium).

### Step 3: preparation of [4-(1-methyl-6-oxo-pyridazin-4-yl)-7-oxo-1,6-diazabicyclo[3.2.1]oct-3-en-6-yl] sulfate sodium salt (Example 19)

Using the procedure described in example 1 (step 7), [4-(1-methyl-6-oxo-pyridazin-4-yl)-7-oxo-1,6-diazabicyclo[3.2.1]oct-3-en-6-yl] sulfate triphenyl-(propenyl)-phosphonium salt (19b) was converted after ion exchange (Dowex sodium form column) into [4-(1-methyl-6-oxo-pyridazin-4-yl)-7-oxo-1,6-diazabicyclo[3.2.1]oct-3-en-6-yl] sulfate sodium salt (Example 19) (7.4 mg, 0.021 mmol, 10% over 2 steps) as a white amorphous solid after lyophilization.
MS *m*/*z* ([M+H]⁺) 329.
MS *m*/*z* ([M-H]⁻) 327.
¹H NMR (400 MHz, D₂O): *δ* (ppm) 3.48 (d, *J* = 11.6 Hz, 1H), 3.77 (s, 3H), 3.79 (dd, *J =* 11.6 Hz, *J* = 2.8 Hz, 1 H), 3.96 (dd, *J* = 19.2 Hz, *J* = 2.8 Hz, 1 H), 4.12 (dd, *J* = 19.2 Hz, *J* = 2.0 Hz, 1H), 4.80 (m, 1 H), 6.53-6.58 (m, 1H), 7.04 (d, *J* = 2.0 Hz, 1H), 8.18 (d, *J* = 2.0 Hz, 1 H).

### Example 20: synthesis of [4-(N-methyl-6-oxo-pyrimidin-4-yl)-7-oxo-1,6-diazabicyclo[3.2.1]oct-3-en-6-yl] sulfate sodium salt

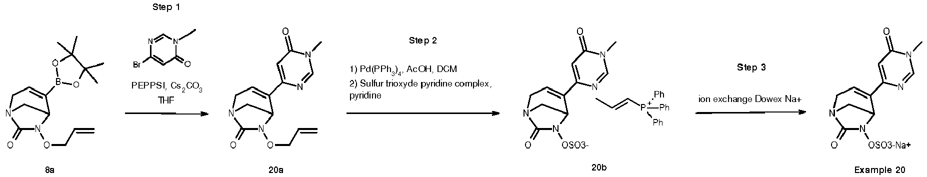

### Step 1: preparation of intermediate 6-allyloxy-4-(N-methyl-6-oxo-pyrimidin-4-yl)-1,6-diazabicyclo[3.2.1]oct-3-en-7-one (20a)

Using the procedure described in example 8 (step 1), the intermediate (6-allyloxy-4-(4,4,5,5-tetramethyl-1,3,2-dioxaborolan-2-yl)-1,6-diazabicyclo[3.2.1]oct-3-en-7-one (8a) (200 mg, 0.653 mmol) is converted into 6-allyloxy-4-(*N*-methyl-6-oxo-pyrimidin-4-yl)-1,6-diazabicyclo[3.2.1]oct-3-en-7-one (20a) (10 mg, 0.035 mmol, 6%) using 4-bromo-*N-*methyl-pyrimidin-6-one (149 mg, 0.790 mmol), PEPPSI catalyst (89 mg, 0.130 mmol) and after purification by chromatography on silica gel (DCM/acetone 100/0 to 40/60).
MS *m*/*z* ([M+H]⁺) 289.
¹H NMR (400 MHz, CDCl₃): *δ* (ppm) 3.10 (d, *J=* 11.2 Hz, 1H), 3.49 (s, 3H), 3.50 (d, *J =* 11.2 Hz, 1 H), 3.62 (dd, *J =* 11.2 Hz, *J* = 3.2 Hz, 1 H), 3.88 (dd, *J* = 19.6 Hz, *J =* 2.0 Hz, 1 H), 4.00 (dd, *J* = 19.6 Hz, *J* = 3.2 Hz, 1 H), 4.36-4.46 (m, 2H), 5.29-5.37 (m, 2H), 5.95-6.06 (m, 1 H), 6.45 (s, 1 H), 6.72 (m, 1 H), 8.04 (s, 1H).

### Step 2: preparation of intermediate [4-(N-methyl-6-oxo-pyrimidin-4-yl)-7-oxo-1,6-diazabicyclo[3.2.1]oct-3-en-6-yl] sulfate triphenyl-(propenyl)-phosphonium salt (20b)

Using the procedure described in example 1 (step 6), the intermediate 6-allyloxy-4-(*N-*methyl-6-oxo-pyrimidin-4-yl)-1,6-diazabicyclo[3.2.1]oct-3-en-7-one (20a) (49 mg, 0.170 mmol) was converted into [4-(*N*-methyl-6-oxo-pyrimidin-4-yl)-7-oxo-1,6-diazabicyclo[3.2.1]oct-3-en-6-yl] sulfate triphenyl-(propenyl)-phosphonium salt (20b) as an amorphous solid after purification by flash chromatography on silica gel (DCM/acetone 50/50 to 0/100).
MS *m*/*z* ([M+H]⁺) 329.
MS *m*/*z* ([M-H]⁻) 327.
MS *m*/*z* ([M+H]⁺) 303 (triphenyl-propenyl-phosphonium).

### Step 3: preparation of [4-(N-methyl-6-oxo-pyrimidin-4-yl)-7-oxo-1,6-diazabicyclo[3.2.1]oct-3-en-6-yl] sulfate sodium salt (Example 20)

Using the procedure described in example 1 (step 7), [4-(*N*-methyl-6-oxo-pyrimidin-4-yl)-7-oxo-1,6-diazabicyclo[3.2.1]oct-3-en-6-yl] sulfate triphenyl-(propenyl)-phosphonium salt (20b) was converted after ion exchange (Dowex sodium form column) into [4-(*N*-methyl-6-oxo-pyrimidin-4-yl)-7-oxo-1,6-diazabicyclo[3.2.1]oct-3-en-6-yl] sulfate sodium salt (Example 20) (2.5 mg, 0.007 mmol, 5% over 2 steps) as a white amorphous solid after lyophilization.
MS *m*/*z* ([M+H]⁺) 329.
MS *m*/*z* ([M-H]⁻) 327.
¹H NMR (400 MHz, D₂O): *δ* (ppm) 3.45 (d, *J =* 11.2 Hz, 1 H), 3.53 (s, 3H), 3.79 (dd, *J =* 11.2/3.2 Hz, 1 H), 3.96 (dd, *J* = 19.6/3.2 Hz, 1H), 4.13 (dd, *J* = 19.6/2.0 Hz, 1H), 4.91 (d, *J* = 2.0 Hz, 1 H), 6.68 (s, 1 H), 6.76-6.80 (m, 1 H), 8.38 (s, 1H).

### Example 21: synthesis of (7-oxo-3-thiazol-2-yl-4-methyl-1,6-diaza-bicyclo[3.2.1]oct-3-en-6-yl) sulfate sodium salt

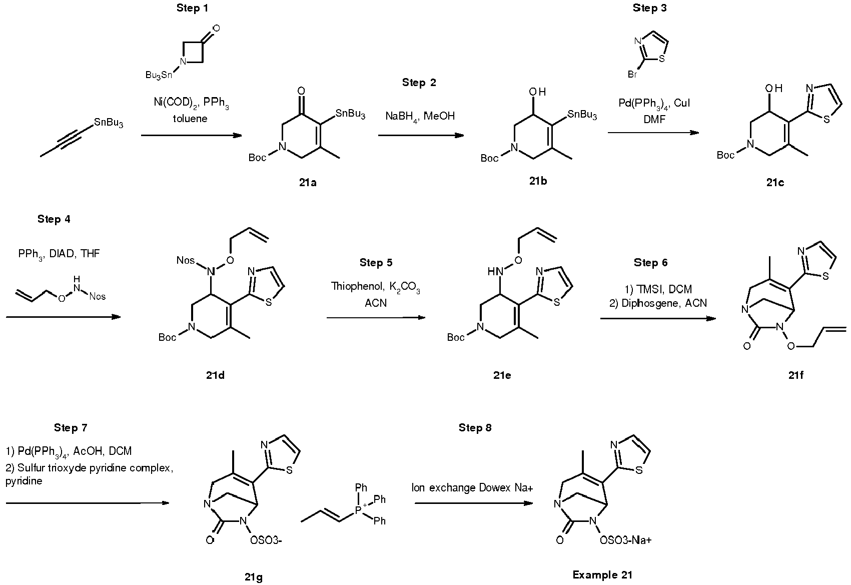

### Step 1: preparation of intermediate tert-butyl 4-methyl-5-oxo-3-(tributylstannyl)-5,6-dihydropyridine-1(2H)-carboxylate (21a)

Under inert atmosphere, Ni(COD)₂ (322 mg, 1.20 mmol) and PPh₃ (613 mg, 2.34 mmol) were added to a solution of 3-Boc-azetidinone (4 g, 23.36 mmol) and tributyl(prop-1-ynyl)stannane (8.9 g , 26.87 mmol) in degassed toluene (140 mL). The reaction mixture was stirred at 60 °C for 2 h, concentrated *in vacuo,* and purified by flash chromatography on silica gel (petroleum ether/Et₂O 100/0 to 80/20) to provide *tert*-butyl 4-methyl-5-oxo-3-(tributylstannyl)-5,6-dihydropyridine-1(2H)-carboxylate (21 a) (6.45 g, 12.89 mmol, 55%) as a colorless oil.
MS *m*/*z* ([M+Na]⁺) 524.
¹H NMR (400 MHz, CDCl₃): δ (ppm) 0.88-1.47 (m, 36H), 2.01 (t, *J=* 2.5 Hz, 3H), 4.00 (bs, 2H), 4.06 (bs, 2H).

### Step 2: preparation of intermediate tert-butyl 4-methyl-5-hydroxy-3-(tributylstannyl)-5,6-dihydropyridine-1(2H)-carboxylate (21b)

A solution of *tert-*butyl 4-methyl-5-oxo-3-(tributylstannyl)-5,6-dihydropyridine-1(2H)-carboxylate (21a) (3 g, 6.00 mmol) in dry MeOH (50 mL) under inert atmosphere was cooled down to 0°C with an ice bath. NaBH₄ (295 mg, 7.80 mmol) was added by portions over 15 min. The reaction was stirred at 0°C for 1 h. Another portion of NaBH₄ was added to the clear yellow solution (90 mg, 2.40 mmol). After 3 h, the reaction was stopped, concentrated to approximatively 20 mL under reduced pressure. The resulting solution was diluted with EtOAc (100 mL), washed with brine (30 mL). The organic phase was dried over sodium sulfate, filtered and concentrated under reduced pressure. The crude residue was purified by flash chromatography on silica gel (heptane/EtOAc 100/0 to 70/30) to give desired *tert-butyl* 4-methyl-5-hydroxy-3-(tributylstannyl)-5,6-dihydropyridine-1(2H)-carboxylate (21 b) (1.62 g, 3.23 mmol, 54%) as a white solid and a clean fraction of recovered starting keto derivative (21 a) (703 mg, 1.41 mmol, 23%).
MS *m*/*z* ([M+Na]⁺) 526.

### Step 3: preparation of intermediate tert-butyl 4-methyl-3-hydroxy-5-(thiazol-2-yl)-5,6-dihydropyridine-1(2H)-carboxylate (21c)

Two 25 mL sealed tube were charged with *tert*-butyl 4-methyl-5-hydroxy-3-(tributylstannyl)-5,6-dihydropyridine-1(2H)-carboxylate (21 b) (810 mg, 1.613 mmol) and diluted with DMF (16 mL). In each tube, 2-bromothiazole (397 mg, 2.42 mmol) was added followed by copper iodide (307 mg, 1.61 mmol). Both suspensions were degassed with argon and Pd(PPh₃)₄ (186 mg, 0.161 mmol) was added. The reactions were stirred at 50°C under argon until complete conversion of starting material. The resulting clear green solutions were combined, concentrated under reduced pressure. The residue was taken up in DCM (20 mL), filtered on PTFE 0.45 µm. The filtrate was concentrated under reduced pressure and purified by flash chromatography on silica gel (heptane/EtOAc 100/0 to 30/70) then on reverse phase (water/ACN 98/2 to 40/60) to give desired intermediate *tert*-butyl 4-methyl-5-hydroxy-3-(thiazol-2-yl)-5,6-dihydropyridine-1 (2H)-carboxylate (21 c) (348 mg, 1.17 mmol, 36%).
MS *m*/*z* ([M+H]⁺) 297.

### Step 4: preparation of intermediate tert-butyl 4-methyl-3-[allyloxy-(2-nitrobenzenesulfonyl)-amino]-5-(thiazol-2-yl)-5,6-dihydropyridine-1(2H)-carboxylate (21d)

Using the procedure described in example 1 (step 2), the intermediate *tert*-butyl 4-methyl-5-hydroxy-3-(thiazol-2-yl)-5,6-dihydropyridine-1(2*H*)-carboxylate (21c) (348 mg, 1.17 mmol) was converted into *tert-butyl* 4-methyl-5-[allyloxy-(2-nitro-benzenesulfonyl)-amino]-3-(thiazol-2-yl)-5,6-dihydropyridine-1(2*H*)-carboxylate (21d) (351 mg, 0.654 mmol, 56%) as a pale yellow oil after purification by flash chromatography on silica gel (heptane/EtOAc 100/0 to 50/50).
MS *m*/*z* ([M+H]⁺) 537.

### Step 5: preparation of intermediate tert-butyl 4-methyl-3-allyloxyamino-5-(thiazol-2-yl)-5,6-dihydropyridine-1(2H)-carboxylate (21e)

Using the procedure described in example 1 (step 3), the intermediate *tert*-butyl 4-methyl-5-[allyloxy-(2-nitro-benzenesulfonyl)-amino]-3-(thiazol-2-yl)-5,6-dihydropyridine-1(2*H*)-carboxylate (21 d) (533 mg, 0.933 mmol) was converted into *tert-butyl* 4-methyl-5-allyloxyamino-3-(thiazol-2-yl)-5,6-dihydropyridine-1(2*H*)-carboxylate (21 e) (280 mg, 0.797 mmol, 80%) after purification by flash chromatography on silica gel (toluene/Et₂O 90/10 to 20/80).
MS *m*/*z* ([M+H]⁺) 352.

### Step 6: preparation of intermediate 3-allyloxyamino-4-methyl-5-thiazol-2-yl-5,6-dihydropyridine (21 f)

Using the procedure described in example 1 (step 4), the intermediate *tert-*butyl 4-methyl-3-allyloxyamino-5-(thiazol-2-yl)-5,6-dihydropyridine-1(2*H*)-carboxylate (21 e) (280 mg, 0.797 mmol) was converted into 6-allyloxy-4-methyl-3-thiazol-2-yl-1,6-diazabicyclo[3.2.1]oct-3-en-7-one (21f) as a pale yellow solid (140 mg, 0.505 mmol, 63% over 2 steps) after purification by flash chromatography on silica gel (heptane/EtOAc 100/0 to 0/100).
MS *m*/*z* ([M+H]⁺) 278.
¹H NMR (400 MHz, CDCl₃): *δ* (ppm) 2.01 (s, 3H), 3.22 (d, *J* = 10.6 Hz, 1H), 3.62 (dd, *J* = 10.7/2.5 Hz, 1 H), 3.81 (dd, *J* = 18.2/1.0 Hz, 1 H), 3.91 (d, *J =* 18.2 Hz, 1 H), 4.37-4.49 (m, 2H), 4.96 (d, *J* = 3.1 Hz, 1 H), 5.25-5.35 (m, 2H), 5.91-6.04 (m, 1 H), 7.42 (d, *J =* 3.3 Hz, 1 H), 7.87 (d, *J* = 3.3 Hz, 1H).

### Step 7: preparation of intermediate (7-oxo-4-methyl-3-thiazol-2-yl-1,6-diazabicyclo[3.2.1]oct-3-en-6-yl) sulfate triphenyl-(propenyl)-phosphonium salt (21g)

Using the procedure described in example 1 (step 6), the intermediate 6-allyloxy-4-methyl-3-thiazol-2-yl-1,6-diaza-bicyclo[3.2.1]oct-3-en-7-one (21f) (140 mg, 0.505 mmol) was converted into (7-oxo-4-methyl-3-thiazol-2-yl-1,6-diaza-bicyclo[3.2.1]oct-3-en-6-yl) sulfate triphenyl-(propenyl)-phosphonium salt (21 g) (245mg) after purification by flash chromatography on silica gel (DCM/acetone 100/0 to 25/75).
MS *m*/*z* ([M+H]⁺) 317.
MS *m*/*z* ([M-H]⁻) 316.
MS *m*/*z* ([M+H]⁺) 303 (triphenyl-propenyl-phosphonium).

### Step 8: preparation of (7-oxo-4-methyl-3-thiazol-2-yl-1,6-diaza-bicyclo[3.2.1]oct-3-en-6-yl) sulfate sodium salt (Example 21)

Using the procedure described in example 1 (step 7), (7-oxo-4-methyl-3-thiazol-2-yl-1,6-diaza-bicyclo[3.2.1]oct-3-en-6-yl) sulfate triphenyl-(propenyl)-phosphonium salt (21 g) (245 mg) was converted after ion exchange (Dowex sodium form column) into (7-oxo-4-methyl-3-thiazol-2-yl-1,6-diaza-bicyclo[3.2.1]oct-3-en-6-yl) sulfate sodium salt (Example 21) (89.8 mg, 0.265 mmol, 55% over 2 steps) as a white amorphous solid after lyophilization.
MS *m*/*z* ([M-H]⁻) 316.
¹H NMR (400 MHz, D₂O): *δ* (ppm) 1.89 (s, 3H), 3.49 (d, *J =* 11.0 Hz, 1 H), 3.75 (dd, *J* = 11.0/3.0 Hz, 1 H), 3.83 (d, *J =* 18.6 Hz, 1H), 4.04 (d, *J =* 18.6 Hz, 1H), 4.88 (d, *J* = 3.1 Hz, 1 H), 7.64 (d, *J* = 3.3 Hz, 1 H), 7.83 (d, *J* = 3.3 Hz, 1H).

### Example 22: biological activity

### Method 1: β-lactamase inhibitory activity, determination of IC₅₀ table 1)

Enzyme activity was monitored by spectrophotometric measurement of nitrocefin (NCF - TOKU-E, N005) hydrolysis at 485nm, at room temperature and in assay buffer A: 100mM Phosphate pH7, 2% glycerol and 0.1 mg/ mL Bovine serum albumin (Sigma, B4287). Enzymes were cloned in *E. coli* expression vector, expressed and purified in house using classical procedures. To a transparent polystyrene plate (Corning, 3628) were added in each well 5µL DMSO or inhibitor dilutions in DMSO and 80µL enzyme in buffer A. Plates were immediately read at 485nm in a microplate spectrophotometer (BioTek, PowerWave HT) to enable background subtraction. After 30min of pre-incubation at room temperature, 15µL of NCF (200µM final) were finally added in each well. Final enzyme concentrations were 0.1 nM (TEM-1), 0.4nM (CTX-M-15), 1 nM (KPC-2), 0.2nM (P99 AmpC) and 0.3nM (OXA-48). After 20min incubation at room temperature, plates were once again read at 485nm. Enzyme activity was obtained by subtracting the final signal by the background, and was converted to enzyme inhibition using non inhibited wells. IC₅₀ curves were fitted to a classical Langmuir equilibrium model with Hill slope using XLFIT (IDBS).

**Table 1: IC₅₀ (nM) for β-lactamase Inhibitory Activity**

| | IC₅₀ (nM) | | | | |
|---|---|---|---|---|---|
| | TEM-1 | CTX-M-15 | KPC-2 | P99 AmpC | OXA-48 |
| Example 1 | 13 | 26 | 770 | 6100 | 8.9 |
| Example 2 | 2.7 | 33 | 1200 | 670 | 2.8 |
| Example 3 | 3.4 | 2.8 | 290 | 770 | 3.5 |
| Example 4 | 2.9 | 7.5 | 390 | 1500 | 2.9 |
| Example 5 | 11 | 6.9 | 270 | 670 | 5.7 |
| Example 6 | 7.3 | 1.7 | 460 | 1800 | 3.7 |
| Example 7 | 12 | 13 | 150 | 1700 | 4.9 |
| Example 8 | 8.2 | 12 | 170 | 1900 | 2.2 |
| Example 9 | 7.9 | 61 | 15 | 1400 | 3.9 |
| Example 10 | 7.7 | 8.3 | 130 | 1100 | 6.1 |
| Example 11 | 27 | 29 | 1800 | 8900 | 5.1 |
| Example 12 | 3.1 | 5.7 | 310 | 1400 | 3.2 |
| Example 13 | 6.1 | 6.0 | 63 | 2800 | 2.3 |
| Example 14 | 1.6 | 2.7 | 52 | 890 | 1.4 |
| Example 15 | 9.9 | 6.3 | 110 | 2000 | 7.9 |
| Example 16 | 56 | 100 | 4500 | 7300 | 10 |
| Example 17 | 1.2 | 5.7 | 130 | 2300 | 3.9 |
| Example 18 | 2.7 | 17 | 1800 | 230 | 20 |
| Example 19 | 4 | 5.4 | 46 | 940 | 4.7 |
| Example 20 | 1 | 3.5 | 87 | 1900 | 2.2 |
| Example 21 | 21 | 14 | 3500 | 180 | 1 |

### Method 2: Synergy with ceftazidime against bacterial isolates (table 2 and table 3)

Compounds of the present invention were assessed against genotyped bacterial strains in combination with the β-lactam ceftazidime. In the assays, MICs of ceftazidime at fixed concentrations of said compounds were determined by the broth microdilution method according to the Clinical Laboratory Standards Institute (CLSI - M7-A7). Briefly, compounds according to the invention and ceftazidime dilutions were prepared in DMSO and spotted (1µL each) on sterile polystyrene plates (Corning, 3788). Log phase bacterial suspensions were adjusted to a final density of 5x10⁵ cfu/ mL in cation-adjusted Mueller-Hinton broth (Beckton-Dickinson) and added to each well (98µL). Microplates were incubated for 16-20 h at 35 °C in ambient air. The MIC of ceftazidime at each compound concentration was defined as the lowest concentration of ceftazidime that prevented bacterial growth as read by visual inspection.

**Table 2: Bacterial isolate**

| ID | Organism | Enzymes |
|---|---|---|
| 1 | *E. coli* | TEM-1 / SHV-12 / CTX-M-15 / OXA-1 |

**Table 3: Synergy MIC assay**

| | MIC (µg/mL) |
|---|---|
| | *E. coli* TEM-1 / SHV-12 / CTX-M-15 / OXA-1 |
| Ceftazidime | 128 |
| Ceftazidime + compound example 1 (4µg/mL) | 0.25 |
| Ceftazidime + compound example 2 (4µg/mL) | 1 |
| Ceftazidime + compound example 3 (4µg/mL) | <=0.125 |
| Ceftazidime + compound example 4 (4µg/mL) | <=0.125 |
| Ceftazidime + compound example 5 (4µg/mL) | <=0.125 |
| Ceftazidime + compound example 6 (4µg/mL) | <0.5 |
| Ceftazidime + compound example 7 (4µg/mL) | <=0.125 |
| Ceftazidime + compound example 8 (4µg/mL) | <=0.125 |
| Ceftazidime + compound example 9 (4µg/mL) | 0.25 |
| Ceftazidime + compound example 10 (4µg/mL) | <=0.125 |
| Ceftazidime + compound example 11 (4µg/mL) | 2 |
| Ceftazidime + compound example 12 (4µg/mL) | <=0.125 |
| Ceftazidime + compound example 13 (4µg/mL) | <=0.125 |
| Ceftazidime + compound example 14 (4µg/mL) | <0.125 |
| Ceftazidime + compound example 15 (4µg/mL) | <=0.125 |
| Ceftazidime + compound example 16 (4µg/mL) | 4 |
| Ceftazidime + compound example 17 (4µg/mL) | <=0.125 |
| Ceftazidime + compound example 18 (4µg/mL) | 1 |
| Ceftazidime + compound example 19 (4µg/mL) | <=0.125 |
| Ceftazidime + compound example 20 (4µg/mL) | <=0.125 |
| Ceftazidime + compound example 21 (4µg/mL) | 4 |

## Claims

1. A compound selected from the group consisting of a compound of formula (I) wherein R¹ represents A and R² represents B and a compound of formula (I) wherein R¹ represents B and R² represents A 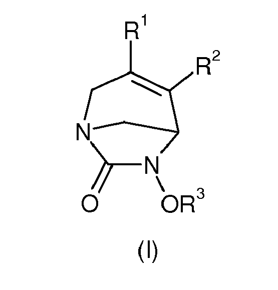 wherein
• A, unsubstituted or substituted by one or more T¹, represents a saturated, partially or totally unsaturated or aromatic 4- to 10-membered heterocycle ;
• B, represents a hydrogen atom ; a fluorine atom ; -(CH₂)ₘOQ¹ ; -(CH₂)ₘ-CN ; - (CH₂)m-OC(O)Q¹ ; -(CH₂)ₘ-C(O)OQ¹ ; (CH₂)ₘ-OC(O)OQ¹ ; (CH₂)ₘ-OC(O)NQ¹Q²; - (CH₂)ₘ-C(O)NQ¹Q² ; (CH₂)ₘ-C(O)ONQ¹Q² ; (CH₂)ₘ-C(O)NQ¹OQ² ; -(CH₂)ₘ-C(O)NQ¹-NQ¹Q² ; -(CH₂)ₙ-NQ¹C(O)Q²; -(CH₂)ₙ-NQ¹S(O)₂NQ¹Q² ; -(CH₂)ₙ-NQ¹S(O)₂Q² ; -(CH₂)ₙ-NQ¹C(O)OQ² ; -(CH₂)ₙ-NQ¹C(O)NQ¹Q² ; -(CH₂)ₙ-NQ¹Q² ; - (CH₂)ₙ-NH-C(NHQ³)=NQ⁴; -(CH₂)ₙ-NH-CH=NQ³ ; -(CH₂)ₘ-C(NHQ³)=NQ⁴ ; or an unsubstituted or substituted by one or more T², (C₁-C₃)-alkyl ; (C₁-C₃)-fluoroalkyl ; O-(C₁-C₃)-fluoroalkyl ; -(CH₂)ₘ-(C₃-C₆)-cycloalkyl ; -(CH₂)ₘ-(C₃-C₆)-cyclofluoroalkyl ;
• R³ represents -SO₃H, -CFHCOOH or -CF₂COOH;
• Q¹ and Q², identical or different, independently represent a hydrogen atom ;-(CH₂)ₚ-NHQ³ ; -(CH₂)ₚ-NH-C(NHQ³)=NQ⁴ ; -(CH₂)ₚ-NH-CH=NQ³ ; (CH₂)ₙ-C(NHQ³)=NQ⁴ ; -(CH₂)ₚ-OQ³ ; -(CH₂)ₙ-CONHQ³ ; or
an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or
Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ;
• Q³ and Q⁴, identical or different, independently represent a hydrogen atom or (C₁-C₃)-alkyl;
• T¹, identical or different, independently represents a fluorine atom ; -(CH₂)ₘOQ¹ ; -(CH₂)ₘ-CN ; -(CH₂)ₘ-OC(O)Q¹ ; -(CH₂)ₘ-C(O)OQ¹ ; -(CH₂)ₘ-OC(O)OQ¹ ; -(CH₂)ₘ-OC(O)NQ¹Q² ; (CH₂)ₘ-C(O)NQ¹Q² ; -(CH₂)ₘ-C(O)ONQ¹Q² ; - (CH₂)ₘ-C(O)NQ¹OQ² ; -(CH₂)ₘ-C(O)NQ¹-NQ1Q² ; -(CH₂)ₘ-NQ¹C(O)Q² **;** -(CH₂)ₘ-NQ¹S(O)₂NQ¹Q² ; -(CH₂)ₘ-NQ¹S(O)₂Q² ; -(CH₂)ₘ-NQ¹C(O)OQ² ; -(CH₂)ₘ-NQ¹C(O)NQ¹Q² ; -(CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(CH₂)ₙ-CN ; -(X)-(CH₂)ₚ-OC(O)Q¹ ; -(X)-(CH₂)ₙ-C(O)OQ¹ ; -(X)-(CH₂)ₚ-OC(O)OQ¹ ; -(X)-(CH₂)ₚ-OC(O)NQ¹Q² ; -(X)-(CH₂)ₙ-C(O)NQ¹Q² ; -(X)-(CH₂)-C(O)ONQ¹Q² ; -(X)-(CH₂)ₙ-C(O)NQ¹OQ² ; -(X)-(CH₂)ₙ-C(O)NQ¹-NQ¹Q² ; -(X)-(CH₂)ₚ-NQ¹C(O)Q² ; -(X)-(CH₂)ₚ-NQ¹S(O)₂NQ¹Q² ; -(X)-(CH₂)ₚ-NQ¹S(O)₂Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)ₚ-NH-CH=NQ³ ; -(X)-(CH₂)ₙ-C(NHQ³)=NQ⁴ ; or
T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; O-(C₁-C₃)-fluoroalkyl ; -(CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ;
• T², identical or different, independently represents -OH ; -NH₂ ; -CONH₂ ;
• m, identical or different, independently represents 0, 1, 2 or 3 ;
• n, identical or different, independently represents 1, 2 or 3 ;
• p, identical or different, independently represents 2 or 3 ;
• X, identical or different, independently represents O ; S ; S(O) ; S(O)₂ or N(Q³);
wherein
• any carbon atom present within a group selected from alkyl, cycloalkyl, fluoroalkyl, cyclofluoroalkyl and heterocycle can be oxidized to form a C=O group ;
• any sulphur atom present within a heterocycle can be oxidized to form a S=O group or a S(O)₂ group ;
• any nitrogen atom present within a heterocycle or present within group wherein it is trisubstituted thus forming a tertiary amino group, can be further quaternized by a methyl group ;
and a racemate, an enantiomer, a diastereoisomer, a geometric isomer or a pharmaceutically acceptable salt thereof.

2. A compound according to claim 1 wherein A represents
• an unsubstituted or substituted by one or more T¹, saturated, partially or totally unsaturated or aromatic 4-, 5- or 6-membered heterocycle comprising at least one nitrogen atom ; or
• an unsubstituted or substituted by one or more T¹, saturated, partially or totally unsaturated or aromatic 4-, 5- or 6-membered heterocycle comprising at least one nitrogen atom and at least one further heteroatom or heteroatomic group selected from O, S, S(O), S(O)₂ and N.

3. A compound according to one of claims 1 to 2 selected from compounds of formulae (I*), (A), (B), (A*) and (B*) 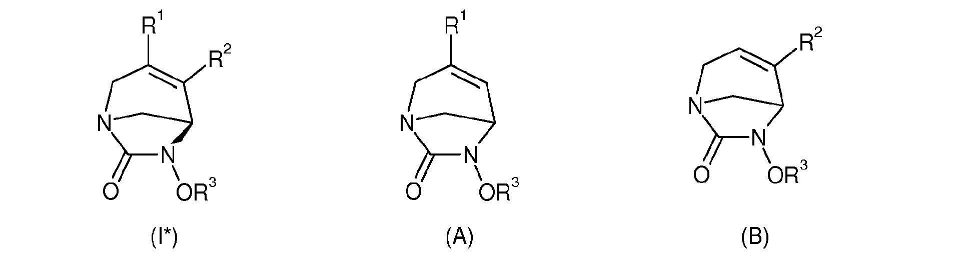 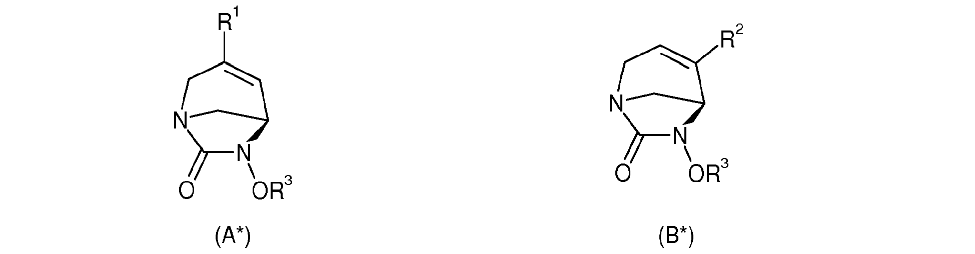 wherein R¹, R² and R³ are defined according to claims 1 or 2.

4. A compound according to one of claims 1 and 2 selected from compounds of formulae (C) and (C*) 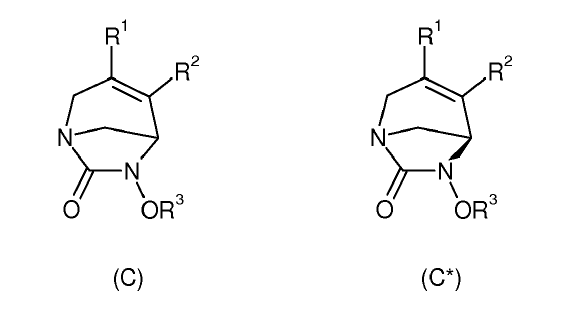 wherein R¹, R² and R³ are defined according to claims 1 or 2 provided that B does not represent a hydrogen atom.

5. A pharmaceutical composition comprising at least one compound according to one of claims 1 to 4 and a pharmaceutically acceptable excipient.

6. A pharmaceutical composition according to claim 5 further comprising at least one compound selected from an antibacterial compound, preferably a β-lactam compound.

7. A pharmaceutical composition according to one of claims 5 and 6 comprising
• a single compound according to one of claims 1 to 4 ;
• a compound according to one of claims 1 to 4 and one or more antibacterial compound ;
• a compound according to one of claims 1 to 4 and one or more β-lactam compound ;
• a compound according to one of claims 1 to 4, one or more antibacterial compound and one or more β-lactam compound.

8. A pharmaceutical composition according to one of claims 6 and 7 wherein
• the antibacterial compound is selected from aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
• the β-lactam compound is selected from β-lactams and mixtures thereof, preferably penicillin, cephalosporins, penems, carbapenems and monobactam.

9. A kit comprising a pharmaceutical composition according to one of claims 5 to 8 and at least one second composition according to one of claims 5 to 8.

10. A compound or a composition according to one of claims 1 to 9 for its use as a medicine or for its use for treating or preventing a bacterial infection.

11. A compound or a composition according to one of claims 1 to 10 for its use as an antibacterial agent and/or as a β-lactamase inhibitor.

12. A compound or a composition according to claim 11 for its use for treating or preventing a bacterial infection caused by bacteria producing one or more β-lactamase.

13. A compound or a composition according to one of claims 1 to 12 for its use for treating or preventing a bacterial infection caused by a gram-positive bacteria or by gram-negative bacteria, preferably a bacterial infection caused by gram-negative bacteria.

14. A kit according to claim 9 for the treatment or prevention of bacterial infections by its simultaneous, separate or sequential administration to a patient in need thereof.
